# EUROPEAN PATENT SPECIFICATION

(11) **EP 4 162 439 B1**
(45) Date of publication and mention of the grant of the patent: **30.09.2026**
(21) Application number: 21820909.6
(22) Date of filing: 08.06.2021
(51) Int. Cl.: G06T 1/40, G06T 7/00, G06T 7/11

(54) **METHODS AND SYSTEMS FOR DETERMINING AN OBJECT MAP**
VERFAHREN UND SYSTEME ZUR BESTIMMUNG EINER OBJEKTKARTE
PROCÉDÉS ET SYSTÈMES DE DÉTERMINATION D'UNE CARTE D'OBJET

(30) Priority: 08.06.2020 US 202063036280 P
(43) Date of publication of application: 12.04.2023
(73) Proprietor: UNITED STATES GOVERNMENT as represented by THE DEPARTMENT OF VETERANS AFFAIRS, Washington, DC 20420 (US)
(72) Inventor: RODRIGUEZ-DIAZ, Eladio, Washington, DC 20420 (US); SINGH, Satish, Washington, DC 20420 (US)
(74) Representative: CH Kilger Anwaltspartnerschaft mbB
(86) International application number: PCT/US2021/036420
(87) International publication number: WO 2021/252502

(56) References cited:
- WO-A1-2017/201540
- US-A1- 2005 107 691
- US-A1- 2016 217 573
- US-B1- 6 184 935
- RODRIGUEZ-DIAZ ELADIO ET AL: "ARTIFICIAL INTELLIGENCE-AUGMENTED VISUALIZATION WITH REAL TIME HISTOLOGY MAPPING OF COLORECTAL POLYPS", 19 May 2020 (2020-05-19), XP093213384, Retrieved from the Internet <URL:https://www.sciencedirect.com/science/article/pii/S0016508520316176?via%3Dihub>
- COUDRAY NICOLAS ET AL: "Classification and mutation prediction from non-small cell lung cancer histopathology images using deep learning", NATURE MEDICINE, NATURE PUBLISHING GROUP US, NEW YORK, vol. 24, no. 10, 17 September 2018 (2018-09-17), pages 1559 - 1567, XP036608997, ISSN: 1078-8956, [retrieved on 20180917], DOI: 10.1038/S41591-018-0177-5
- ARKADIUSZ GERTYCH ET AL: "Convolutional neural networks can accurately distinguish four histologic growth patterns of lung adenocarcinoma in digital slides", SCIENTIFIC REPORTS, vol. 9, no. 1, 6 February 2019 (2019-02-06), XP055732254, DOI: 10.1038/s41598-018-37638-9
- JOKERST JESSE V., COLE ADAM J., VAN DE SOMPEL DOMINIQUE, GAMBHIR SANJIV S.: "Gold Nanorods for Ovarian Cancer Detection with Photoacoustic Imaging and Resection Guidance via Raman Imaging in Living Mice", ACS NANO, vol. 6, no. 11, 27 November 2012 (2012-11-27), pages 10366 - 10377, XP055883052, Retrieved from the Internet <URL:https://www.ncbi.nlm.nih.gov/pmc/articles/PMC3572720/pdf/nihms437377.pdf> [retrieved on 20210810]
- WANG YU-SHUEN, TAI CHIEW-LAN, SORKINE OLGA, LEE TONG-YEE: "Optimized Scale-and-Stretch for Image Resizing", ACM TRANSACTIONS ON GRAPHICS, vol. 27, no. 5, December 2008 (2008-12-01), pages 1 - 8, XP058336179, Retrieved from the Internet <URL:https://dl.acm.org/doi/pdf/10.1145/1457515.1409071> [retrieved on 20210810]
- ZAHANGIR ALOM; THEUS ASPIRAS; TAREK M TAHA; VIJAYAN K ASARI: "Skin Cancer Segmentation and Classification with NABLA-N and Inception Recurrent Residual Convolutional Networks", COMPUTER VISION AND PATTERN RECOGNITION, 25 April 2019 (2019-04-25), pages 1 - 7, XP081173448, Retrieved from the Internet <URL:https://arxiv.org/abs/1904.11126> [retrieved on 20210810]

## Description

This application claims the benefit of U.S. Provisional Application No. 63/036,280, filed June 8, 2020.

### BACKGROUND

Numerous methods have been devised for the treatment and prevention of colon cancer. Colonoscopy is one such technique used for colon cancer screening and prevention, during which a tiny camera is inserted and guided through the colon to detect and treat polyps-potential precursors to colon cancer. At present, colonoscopic colorectal cancer prevention hinges on treatment of the polyps through the complete removal and histopathological assessment of all detected polyps. This costly and cumbersome practice results in the treatment of large quantities of polyps that have negligible malignant potential, since endoscopists lack an accurate and consistent way of classifying polyps in real-time.

### SUMMARY

The present disclosure relates to the treatment of tissue growths and polyps. It is to be understood that both the following general description and the following detailed description provide only examples and are not restrictive.

As one or more examples, a method for treating tissue may include determining an object in image data based on a first classifier. The method may include determining a boundary of the object based on the object. The method may include determining a plurality of sub-images within the boundary based on the boundary of the object. The method may include determining a predicted disease state for each sub-image of the plurality of sub-images. The method may include determining a predicted disease state for the object based on the predicted disease states for each sub-image of the plurality of sub-images.

Also disclosed is a method that may include determining a first training data set comprising a plurality of images containing a labeled boundary of an object. The method may include training a first classifier configured to determine an object boundary based on the first training data set. The method may include determining a second training data set comprising a plurality of labeled sub-images from within the labeled boundary of the object of each image in the first training data set based on the first training data set. The method may include training a second classifier configured to predict a disease state based on the first training data set. The method may include configuring for an input image the first classifier to output a determined object boundary in the input image and the second classifier to output a predicted disease state for each sub-image of a plurality of sub-images from within the object boundary from the input image.

Also disclosed is a method. The method may include determining an object in image data based on a first classifier. The method may include determining a boundary of the object based on the object. The method may include determining a plurality of sub-images within the boundary based on the boundary of the object. The method may include determining a predicted disease state for each sub-image of the plurality of sub-images. The method may include determining a visual indication of the boundary based on the boundary of the object. The method may include determining a visual indication of the predicted disease state for each sub-image of the plurality of sub-images based on the predicted disease states for each sub-image of the plurality of sub-images. The method may include determining an object map based on the visual indication of the boundary and the visual indications of the predicted disease state for each sub-image of the plurality of sub-images. The method may include outputting the image data and the object map as a composite image.

Also disclosed are methods and systems comprising receiving object classification data wherein the object classification data comprises an indication of a predicted object, an indication of one or more regions of the predicted object, and one or more scores associated with the indication of the predicted object, determining data indicative of a boundary of the predicted object, determining a visual representation associated with each of the one or more regions of the predicted object wherein the visual representation may be based on the indication of the one or more regions of the predicted object and the one or more scores associated with the indication of the one or more regions of the predicted object, determining based on the data indicative of the boundary of the predicted object, and the visual representation associated with each of the one or more regions of the predicted object an object map, and outputting, based on the object map and the image data, a composite image. The methods may further comprise determining, based on the one or more scores associated with the indication of the one or more regions of the predicted object, a color associated with the one or more scores associated with the indication of the one or more regions of the predicted object, wherein determining the visual representation associated with each of the one or more regions of the predicted object comprises assigning the color to the visual representation associated with each of the one or more regions of the predicted object.

Also disclosed are methods comprising receiving, from a machine learning classifier, image data, wherein the image data comprises at least one region and a plurality of image sections, and wherein the at least one region comprises at least one subregion, determining, based on at least one image section of the plurality of image sections, an initial classification associated with the at least one image section, determining, based on at least one additional image section of the plurality of image sections, an additional initial classification associated with the at least one additional image section, determining, determining, based on the initial classification and the additional initial classification, a score, wherein the score is associated with the at least one subregion, determining, based on the score, a visual indication, and outputting, based on the visual indication, an object map.

### BRIEF DESCRIPTION OF THE DRAWINGS

In order to provide understanding techniques described, the figures provide nonlimiting examples in accordance with one or more implementations of the present disclosure, in which:
**FIG. 1A** illustrates an example method for tissue treatment with a composite image in accordance with one or more implementations of the present disclosure.
**FIG. 1B** illustrates an example method for determining an object map in accordance with one or more implementations of the present disclosure.
**FIG. 2** illustrates an example machine learning system in accordance with one or more implementations of the present disclosure.
**FIG. 3A** illustrates an example machine learning method in accordance with one or more implementations of the present disclosure.
**FIG. 3B** illustrates an example application of a predictive model in accordance with one or more implementations of the present disclosure.
**FIG. 4** illustrates an example original image from an endoscope alongside a composite image in accordance with one or more implementations of the present disclosure.
**FIG. 5A** illustrates an example visual indication in accordance with one or more implementations of the present disclosure.
**FIG. 5B** illustrates an example visual indication in accordance with one or more implementations of the present disclosure.
**FIG. 6** illustrates an example method for treatment of tissue in accordance with one or more implementations of the present disclosure.
**FIG. 7** illustrates an example computing environment in which the present methods may be executed in accordance with one or more implementations of the present disclosure.
**FIG. 8** illustrates a decolorized version of **FIG. 4** in accordance with one or more implementations of the present disclosure.
**FIG. 9A** illustrates a training method for semantic segmentation or pixel classification in accordance with one or more implementations of the present disclosure.
**FIG. 9B** illustrates a training method for a region of interest or pixel portion classifier in accordance with one or more implementations of the present disclosure.
**FIG. 10** illustrates a portion of a classification method for classifying a region of interest or pixel portion with a trained classification model in accordance with one or more implementations of the present disclosure.
**FIG. 11** illustrates a portion of a treatment for tissue in accordance with one or more implementations of the present disclosure.
**FIG. 12A** illustrates statistical data of polyp pathology in accordance with one or more implementations of the present disclosure.
**FIG. 12B** illustrates statistical data of computer-aided treatment in accordance with one or more implementations of the present disclosure.
**FIG. 12C** illustrates statistical data of confidence in computer-aided treatment in accordance with one or more implementations of the present disclosure.

### DETAILED DESCRIPTION

As used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another configuration includes from the one particular value and/or to the other particular value. Similarly, when values are expressed as approximations, by use of the antecedent "about," it will be understood that the particular value forms another configuration. It will be further understood that the endpoints of each of the ranges are significant both in relation to the other endpoint, and independently of the other endpoint.

"Optional" or "optionally" means that the subsequently described event or circumstance may or may not occur, and that the description includes cases where said event or circumstance occurs and cases where it does not.

Throughout the description and claims of this specification, the word "comprise" and variations of the word, such as "comprising" and "comprises," means "including but not limited to," and is not intended to exclude, for example, other components, integers or steps. "Exemplary" means "an example of" and is not intended to convey an indication of a preferred or ideal configuration. "Such as" is not used in a restrictive sense, but for explanatory purposes.

It is understood that when combinations, subsets, interactions, groups, etc. of components are described that, while specific reference of each various individual and collective combinations and permutations of these may not be explicitly described, each is specifically contemplated and described herein. This applies to all parts of this application including, but not limited to, steps in described methods. Thus, if there are a variety of additional steps that may be performed it is understood that each of these additional steps may be performed with any specific configuration or combination of configurations of the described methods.

As will be appreciated by one skilled in the art, hardware, software, or a combination of software and hardware may be implemented. Furthermore, a computer program product on a computer-readable storage medium (e.g., non-transitory) having processor-executable instructions (e.g., computer software) embodied in the storage medium. Any suitable computer-readable storage medium may be utilized including hard disks, CD-ROMs, optical storage devices, magnetic storage devices, memresistors, Non-Volatile Random Access Memory (NVRAM), flash memory, or a combination thereof.

Throughout this application reference is made to block diagrams and flowcharts. It will be understood that each block of the block diagrams and flowcharts, and combinations of blocks in the block diagrams and flowcharts, respectively, may be implemented by processor-executable instructions. These processor-executable instructions may be loaded onto a general purpose computer, special purpose computer, or other programmable data processing apparatus to produce a machine, such that the processor-executable instructions which execute on the computer or other programmable data processing apparatus create a device for implementing the functions specified in the flowchart block or blocks.

These processor-executable instructions may also be stored in a computer-readable memory that may direct a computer or other programmable data processing apparatus to function in a particular manner, such that the processor-executable instructions stored in the computer-readable memory produce an article of manufacture including processor-executable instructions for implementing the function specified in the flowchart block or blocks. The processor-executable instructions may also be loaded onto a computer or other programmable data processing apparatus to cause a series of operational steps to be performed on the computer or other programmable apparatus to produce a computer-implemented process such that the processor-executable instructions that execute on the computer or other programmable apparatus provide steps for implementing the functions specified in the flowchart block or blocks.

Blocks of the block diagrams and flowcharts support combinations of devices for performing the specified functions, combinations of steps for performing the specified functions and program instruction means for performing the specified functions. It will also be understood that each block of the block diagrams and flowcharts, and combinations of blocks in the block diagrams and flowcharts, may be implemented by special purpose hardware-based computer systems that perform the specified functions or steps, or combinations of special purpose hardware and computer instructions.

Methods and systems are described for using a machine learning classifier(s) for detection and classification of objects. Machine learning (ML) is a subfield of computer science that gives computers the ability to learn through training without being explicitly programmed. Machine learning platforms include, but are not limited to, deep-learning techniques, naive Bayes classifiers, support vector machines, decision trees, neural networks, and the like.

In an aspect, the machine learning classifiers may process, be trained with, or otherwise operate on, image data. The image data may be generated by photographic method, a CT scan, or any other suitable medical imaging technology. As an example, narrow-band imaging (NBI) may be used to generate image data. NBI is an endoscopic technology that uses blue and green light to illuminate the surface mucosa, which results in the visualization of surface pits and microvessels through enhanced contrast. Interest in the use of NBI for polyp histology has led to formalized consensus criteria that categorizes neoplastic and non-neoplastic polyps.

The image data may comprise a two-dimensional image or a three-dimensional image. For example, three-dimensional (3D) medical image data may comprise a series of CT image slices obtained from a CT scan of an area of a human or animal patient. Each slice is a two-dimensional digital greyscale image of the x-ray absorption of the scanned area. In an example, the intensity value of each pixel may be expressed in Hounsfield units (HU). Sequential slices may be separated by a constant distance along the z direction (i.e. the scan separation axis); for example, by a distance of between 0.5-2.5 mm. Hence, the scan image formed by a plurality of slices may be a three-dimensional (3D) greyscale image, with an overall size depending on the area and number of slices scanned. Each pixel may then be considered to be a voxel (or volumetric pixel) in three-dimensional space.

In an example, a first ML classifier may be trained using training data (e.g., image data) to configure the first ML classifier to predict/classify images to define a boundary of an object or otherwise segment the image. The training data may comprise a plurality of medical images and/or diagnostic data. Diagnostic data may include vital sensory information from the patient (e.g., temperature, heart rate). In an example, the training data may comprise one or more labels associated with an object, pixels, portions, and the like, that have been assigned by someone knowledgeable (e.g., a clinician), a computer-based algorithm, or combinations thereof. The one or more labels may be, for example, binary indications of growth-associated tissue. For example, a clinician may designate individual pixels of image data as growth-associated through a selection program. The clinician or program may designate the rest of the pixels of the image data as statistically or empirically ordinary. In such a way, tissue depicted within the image data is classified as TRUE/FALSE, ZERO/ONE, or some other designation to provide a ground truth for training.

Such training data or training data sets can be encoded into a Bayesian model. The probability distribution for each feature may be modeled using a parameterized form, which is based on the training data set and a training step for the learning of parameters. For example, the Bayesian framework can incorporate a model that takes advantage of the fact that polyps comprise different features, for differences in appearance. Features may be learned in one or more layers of the first ML classifier. The features may include one or more appearance features. The appearance features may comprise image information derived from the medical image. The appearance features may comprise a shape feature derived from the medical image. The appearance features comprise a texture feature derived from the medical image. The features may include one or more shape features, for instance a region of the predicted object or a boundary of the predicted object. The shape features may be defined by coordinates or a position relative to another shape feature, or the like. The features may include one or more anatomical features. Determining the value of an anatomical feature may comprise calculating a centerline a boundary using a boundary distance transform. The image may be divided into sections or units (for example, a pixel). The image may be segmented to identify and/or characterize a region of the image that comprises an object, for instance a polyp.

A second ML classifier may be trained using training data. The training data set for the second ML classifier may be the image data or portions thereof (e.g., sub-image data) to configure the second ML classifier to predict/classify neoplasticity or malignancy. The training data used to train the second ML classifier may be the same as, or different from, the training data used to train the first ML classifier. The training data may comprise a plurality of medical images and/or diagnostic data. The images may be derived according to the first ML classifier. For instance, the first ML classifier may identify sub-images within the image data related to a polyp or growth. In an example, labels for the training data (e.g., sub-images) may have been assigned by a clinician. For example, a benign/malignant classification can be determined for regions/subregions of the predicted object and represented on a per image unit basis (for example, a pixel may comprise a benign section of the predicted object or a malignant section). Likewise, each pixel that comprises a benign/malignant classification is also associated with a confidence score that describes the probability that the benign/malignant classification is correct.

Such, training data can be encoded into a Bayesian model. The probability distribution for each feature may be modelled using a parameterized form, which may be based on the training set data and a training step for the learning of parameters. For example, the Bayesian framework can incorporate a model that takes advantage of the fact that benign and malignant polyps comprise different features, for differences in appearance.

Once trained, new image data may be provided to the first ML classifier and/or the second ML classifier. The output of the first ML classifier and/or the second ML classifier, referred to herein as object classification data, may comprise classified pixels, boundaries, segmentations, sub-image classifications, sub-region classifications, confidence scores, and combinations thereof. For instance, the first ML classifier indicates a boundary of an object (e.g., a polyp) and the second ML classifier outputs a prediction of disease state for a plurality of regions of that polyp. These results are stored as object classification data. The confidence score may be related to an entire image, sub-images, sub-regions, and combinations thereof. In another example, the image data may be used to segment the image on a trained segmentation model, classify the image or portions thereof, and provide a confidence score. In addition, the methods disclosed herein allow the inclusion of features that characterize a particular section or unit or point in the image data with respect to prior medical knowledge. The values of the features may be determined at every image unit (e.g., pixel or voxel) in the medical image or may be determined at a portion of the image (e.g., a region) or for a single unit. For example, determining the value of an appearance feature may simply require an intensity value of a particular pixel to be read from the medical image data, whereas the values of shape features and anatomical features are typically determined by performing one or more calculations based upon the intensity value of pixel (and, possibly, its neighboring pixels).

The object classification data may be used to generate an object map and/or composite image. The object classification data may be used to generate a usable output comprising a composite image of a predicted object within the image data. The image data may lead to two or more types (or layers) of classification; a pixel-wise classification or segmentation defining a boundary, a benign/malignant classification, and for each of the benign malignant/classifications a confidence score. The object classification data that is received comprises two or more layers or two or more classifications of data. The data from the two or more layers is combined to generate the object map, and the object map is overlaid with a photograph of the predicted object to generate the composite image.

The composite image may be used as a guide to direct treatment and reduce errors. Techniques are described for treating polyps and other growths on various tissues. A variety of treatments exist for such growths. As an example, resection may be performed to further analyze the growth for cancer. The costs associated with resection and evaluation can add substantially to the total cost of a colonoscopy. Risk of cancer within a polyp increases as the size of the polyp passes five millimeters. For example, some studies have found nearly all polyps below five millimeters to be benign. As such, removal and analysis may be costly and unnecessary. In contrast, 0-1.5% of six to nine millimeter polyps are cancerous and 2-10% of polyps greater than ten millimeters are cancerous. As such, treatment of polyps may be performed when a high likelihood of cancer is presented. For instance, treatment may include identifying tissue with a high likelihood of cancer and resection of the tissue during an endoscopic procedure. In one example procedure, only polyps less than five millimeters visually predicted to be neoplastic are resected and either discarded or subjected to further analysis. In another, polyps less than five millimeters may be resected with only those visually predicted to be neoplastic being subjected to further analysis.

Although varieties of techniques exist for performing machine learning and resecting tissue, specific improvements to those techniques are disclosed herein. The specific techniques disclosed herein describe a practical implementation that pragmatically reduces the required processing power and training data necessary to accurately predict disease state (e.g., cancerous tissues) from tissue images. Indeed, the provided disclosure reduces the error associated with computer-aided growth detection, reducing costs associated with over-resection and under-resection. As an example, a computer-aided treatment that required resection of every identified growth could raise the risk of medical complications associated with resections. Conversely, the same computer-aided treatment could err in identifying neoplastic tissues and recommend non-resection.

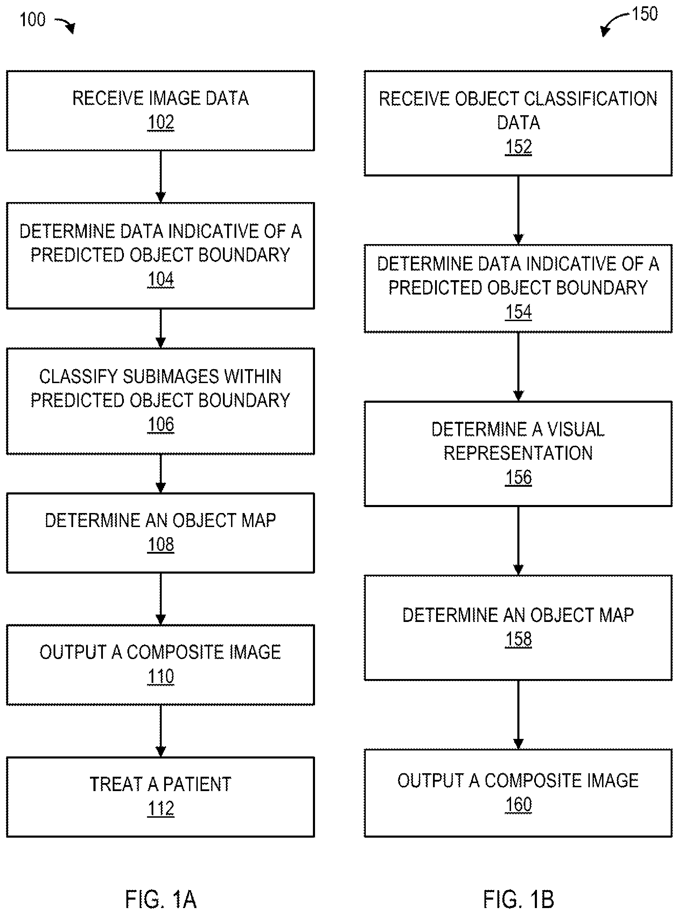

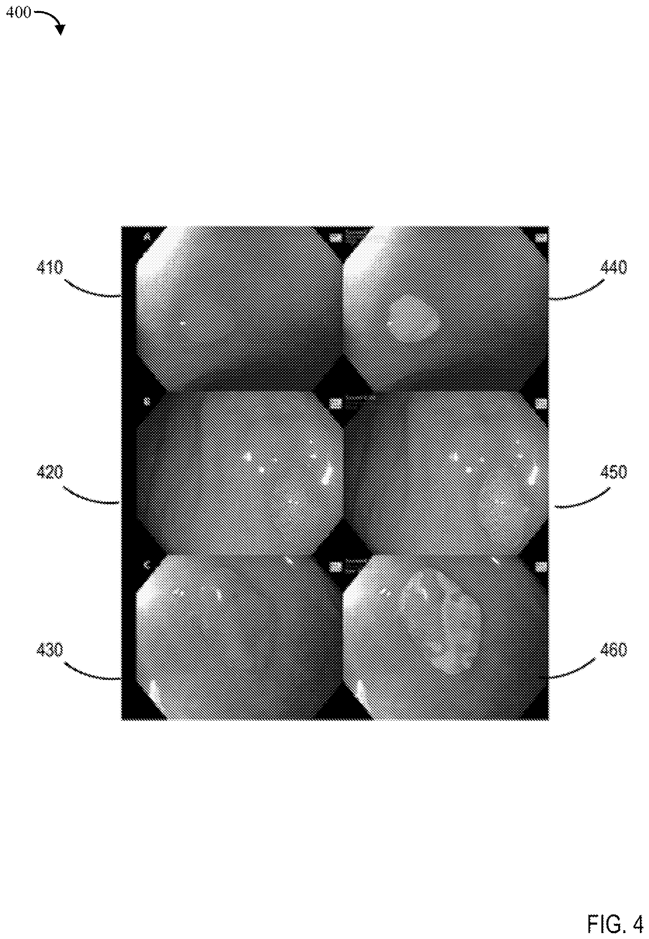

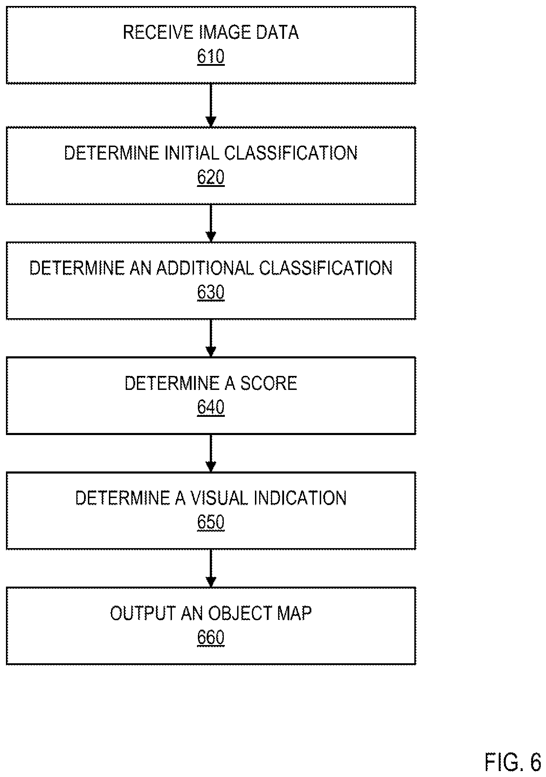

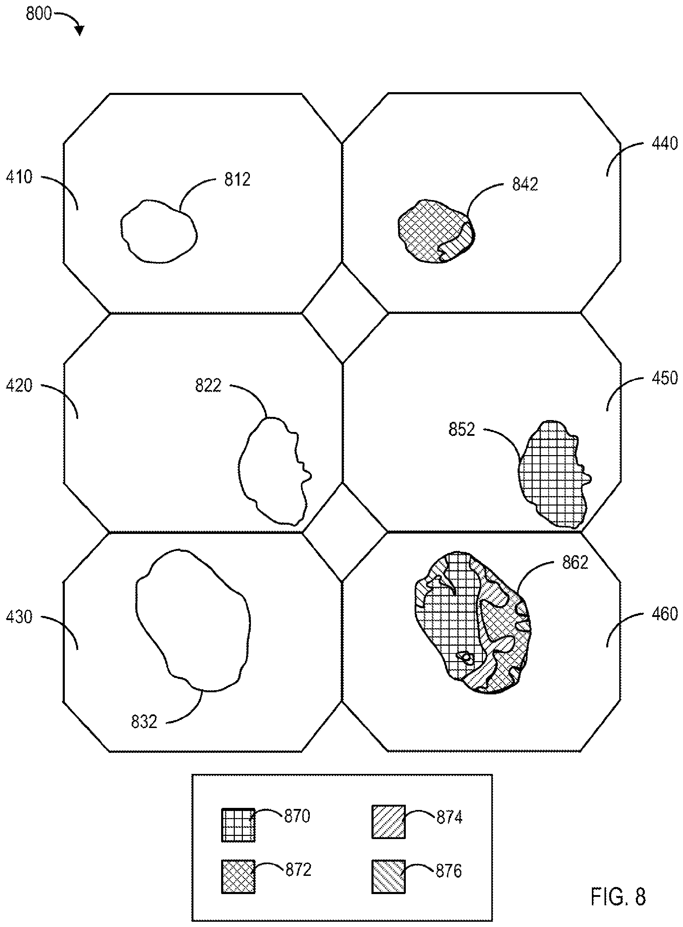

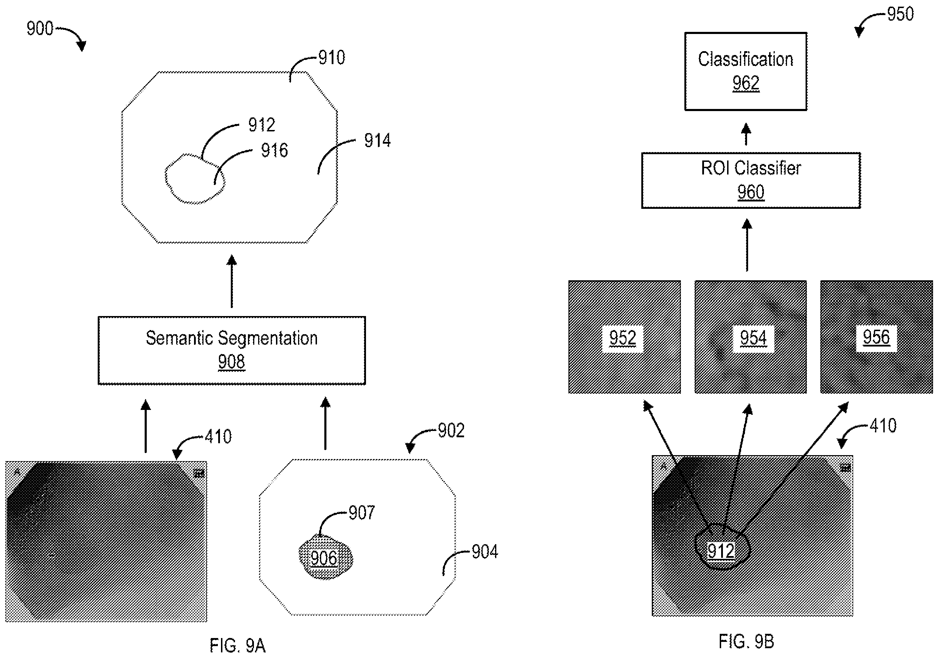

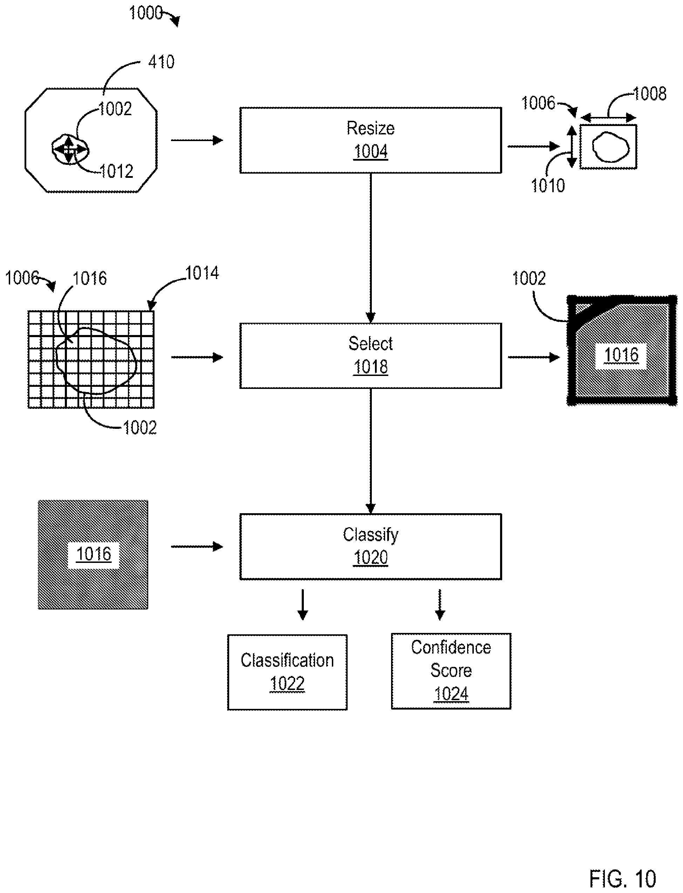

**FIG. 1A** **shows,** an example method **100** for tissue treatment utilizing a composite image in accordance with one or more implementations of the present disclosure. Any of the steps show in method **100** may be combined with any other steps discussed throughout this disclosure. In step **102,** image data may be received by or from an endoscope. The image data an image **412,** as discussed throughout and with regard to **FIG. 4** and **FIG. 8****.** In step **104,** data indicative of a predicted object boundary **1002** may be determined, as discussed throughout and with regard to **FIG. 10****.** As discussed throughout this disclosure and in **FIG. 1****,** **FIG. 6****,** a boundary **1002** may be predicted with regard to an object. Semantic segmentation models, similar to those trained in semantic segmentation model **908** of **FIG. 9A****,** may be used. The semantic segmentation model **908** may be used to segment each image **412** to outline growth and polyp boundaries.

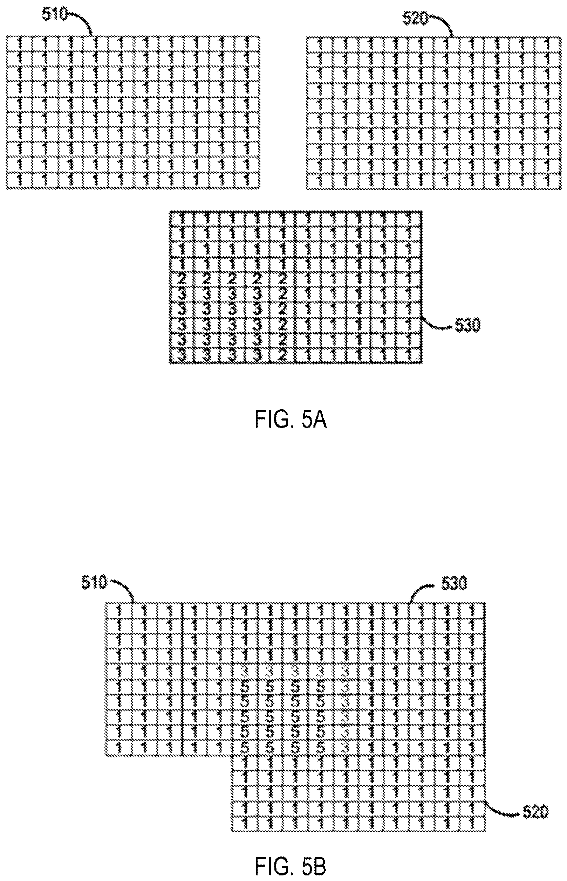

In step **106,** classification of sub-images may be performed. Sub-images may be regions of interest or pixel portions of the image **412,** for example, sub-images may comprise a portion of the image **412** within the boundary of the object. As an example, the sub-images may be a resized image **1006,** as discussed throughout the disclosure including **FIG. 10****.** A classification model similar to ROI classifier **960** may be used, as discussed throughout the disclosure including **FIG. 9B****.** As such, classifications and confidence scores may be generated independently. The sub-image size can be 64 pixels by 64 pixels or another size. In step **108,** an object map may be determined. As discussed throughout this disclosure and with regard to various figures, the object map provides visual representations and indications (e.g., indicia **870, 872, 874, 876).** The object map may be a matrix (e.g., **FIG. 5A** and **FIG. 5B****)** that represent sub-images, regions of interest, or pixel portions with the corresponding scores and hues from the classifier for that sub-image. In step **110,** a composite image **440, 450, 460** may be output or displayed, as shown throughout this disclosure including **FIG. 4** and **FIG. 8****.** After display, an operator, an endoscopist or otherwise, may treat the patient according to the composite image **440, 450, 460,** indicia **870, 872, 874, 876** or other indicia provided on the screen (e.g., aggregate confidence score, aggregate classification) in step **112.** Any of these steps along with other steps throughout this disclosure may be combined, omitted, or otherwise used to treat patients and respective growths.

**FIG. 1B** shows a method **150** for determining an object map and a composite image of steps **108** and **110,** respectively, from **FIG. 1A****.** The method **150** may be carried out by any suitable system, for example, system **700.**

The object classification data may comprise an indication of a predicted object, an indication of one or more regions of the predicted object, a score associated with the indication of the one or more regions of the predicted object, or a score associated with the indication of the predicted object. In an example, the indication of the predicted object may comprise an object identifier such as whether or not the object is a polyp. In an example, the indication of the predicted object may comprise an object classification such as whether or not the object is malignant or benign. In an example, the indication of one or more regions of the predicted object may comprise at least one boundary identification. In an example, the indication of the predicted object may comprise an indication of a boundary of the object which distinguishes the object from its surroundings. For example, the indication of the object may distinguish polyp tissue from non-polyp tissue. The indication may be associated with each of the image sections (e.g., pixels or voxels) or a portion of the image sections.

In an embodiment, the object classification data may be received from a machine learning classifier (e.g., a predictive model) which may the data in any number of ways. Receiving object classification data at step **152** may comprise downloading/obtaining/receiving the object classification data. The object classification data may originate from various sources, including live feeds or streams, databases, live-video, real-time images, other image data and the like for classification. The data may comprise data sets associated with different conditions (e.g., colon cancer, anal cancer, melanoma, breast cancer, lung cancer, ovarian cancer, etc.) and may be generated from various data types and/or platforms.

Determining data indicative of a boundary of the predicted object at step **154** may comprise various implementations that define a boundary of the predicted object from the object classification data, other information, and combinations thereof. The boundary of the predicted object may be processed by applying analysis to determine a visual representation, as will be described and indicated at step **156.** In this manner, one or more boundaries may be generated. The data indicative of a boundary of the predicted object may be used to identify polyp candidates. Determining data indicative of a boundary of the predicted object at step **154** may comprise determining a pixel matrix in the object classification data. The pixel matrix may designate polyp pixels and non-polyp pixels, as an example. Determining data indicative of a boundary of the predicted object at step **154** may comprise determining a mathematical description of an outline of an object (e.g., a formula). The formula may separate polyp pixels and non-polyp pixels with a line or another geometry. Determining data indicative of a boundary of the predicted object at step **154** may comprise determining one or more coordinates for an outline of an object. The one or more coordinates may form a line or another geometry to separate polyp pixels and non-polyp pixels.

Determining a visual representation at **156** may comprise determining an indication (e.g., a score or value) associated with a region of the predicted object. In an example, the indication may comprise an object class (e.g., polyp, non-polyp, etc...). In an example, the visual representation may be associated with a score or a numerical value associated with region. For example, the numerical value may be binary with a 0 indicating a benign region and 1 indicating a malignant. In another example, the numerical value may comprise a probability such as a likelihood or a percentage. The probability may be represented by a numerical value on a scale, for instance between 0 and 1, or 1 and 5, or any other range. A specific example may comprise determining that the region or portion of pixels of the image associated with tissue is classified into one of two categories (e.g., good or bad, zero or one) according to disease state. Other categories may be used. Determining a visual representation associated with regions of the predicted object at step **156** may comprise determining for each sub-image indicated in the object classification data, a predicted disease state and determining a color associated with the disease state. As an example, one predicted disease state may be associated with the color red, and another predicted disease state may be associated with the color green.

The probability may represent a predictive value as to whether or not a region of an object possesses a certain quality, for instance, whether or not a region of an object is malignant or benign. As an example, the certain quality may adjust the redness associated with the predicted disease state.

In an example, the probability may be determined using any statistical method such as, for example, Bayes' law to determine and in some cases combine probability distributions and generate a probability map. In an example, as will be explained below, combining maps may comprise overlaying probability maps wherein each section of an image comprises a probability.

In an example, the image data may comprise a pixel or voxel associated with a probability. In an example, the visual representation may comprise color indicators. For instance, a region of the predicted object may be associated with the color red, to indicate a high confidence that the region is malignant. A different region of the predicted object may be associated with the color green, to indicate a high confidence that region is benign. A different region of the predicted object may be associated with the color yellow, to indicate a low confidence as to whether or not the region is benign or malignant. In an example, determining a score such as a probability comprises calculating the value of a Gaussian function at a particular image unit, wherein the Gaussian function models the probability distribution of that feature. The Gaussian function may model the probability distribution of a feature that characterizes the section of the medical image, and wherein calculating the value of the Gaussian function includes: (i) treating a mean value in the Gaussian function as a fixed value; or (ii) calculating a mean value in the Gaussian function as a function of the size of a previously-detected object.

In an example, the indication of the predicted object may comprise coordinates, for instance coordinates in an x-y plane or an x-y-z volume. The coordinates may be associated with a region of the object. A region of the predicted object may be associated with the coordinates of a plan or volume.

At step **158,** an object map may be determined. For instance, an object map may comprise at least one visual representation. In an example, an object map may comprise at least one visual representation of an object with indications as to whether or not regions of the object possess a given quality, for instance a prediction as to whether or not a region of a polyp is benign or malignant. Determining an object map at step **158** may comprise plotting on an overlay image, a visual representation of the boundary and the visual representations of the regions of the predicted object. Determining an object map at step **158** may comprise altering a color composition of the pixels within the visual representations of the regions of the predicted object.

At step **160,** a composite image may be output. The composite image may comprise at least one image of the predicted object, for example the polyp, and at least one visual representation associated with a region of the object, for example a prediction that a region of the polyp is malignant or benign (as seen in **FIG. 4****).** Outputting the composite image may comprise outputting a combination of the image and the overlay image. Outputting the composite image may comprise outputting the adjusted color composition of the image.

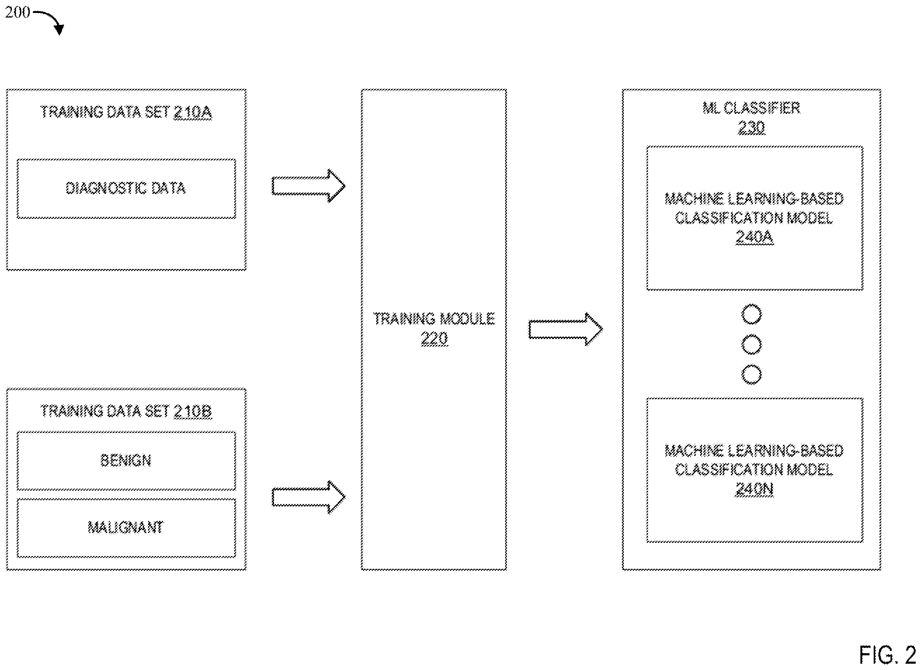

As shown in **FIG. 2****,** a system **200** is described herein that is configured to use machine learning techniques to train, based on an analysis of one or more training data sets **210A-210B** by a training module **220,** at least one machine learning-based classifier **230** that is configured to classify baseline data as being associated with a diagnosis, pathology or histology. In an example, the training data set **210A** (e.g., the data) may comprise curated data from one or more studies. In an example, the training data set **210A** may comprise only curated data or only a portion of the curated data. In an example, the training data set **210B** (e.g., the in-treatment data) may comprise data. The training data set **210B** includes thousands of images from segmented polyps with annotations (e.g., neoplasticity, malignancy). In an example, the training data set **210B** may comprise only the labeled data or only a portion of the labeled data. The labels may comprise benign and malignant labels.

Data may be randomly assigned to the training data set **210B** or a testing data set. In some implementations, the assignment of data to a training data set or a testing data set may not be completely random. In this case, one or more criteria may be used during the assignment, such as ensuring that similar numbers of diagnosis with different benign/malignant statuses are in each of the training and testing data sets. In general, any suitable method may be used to assign the data to the training or testing data sets, while ensuring that the distributions of benign/malignant statuses are somewhat similar in the training data set and the testing data set. In an example, 75% of the labeled baseline data may be assigned to the training data set **210B** and 25% of the labeled baseline data may be assigned to the test data set.

In an example, the training module **220** may train the machine learning-based classifier **230** by extracting a feature set from the first data (e.g., the curated case agnostic data) in the training data set **210A** according to one or more feature selection techniques. In an example, the training module **220** may further define the feature set obtained from the training data set **210A** by applying one or more feature selection techniques to the second data (e.g., the labeled baseline data) in the training data set **210B** that includes statistically significant features of positive examples (e.g., malignant) and statistically significant features of negative examples (e.g., benign).

In an example, the training module **220** may extract a feature set from the training data set **210A** and/or the training data set **210B** in a variety of ways. The training module **220** may perform feature extraction multiple times, each time using a different feature-extraction technique. In an example, the feature sets generated using the different techniques may each be used to generate different machine learning-based classification models **240.** In an example, the feature set with the highest quality metrics may be selected for use in training. The training module **220** may use the feature set(s) to build one or more machine learning-based classification models **240A**-240N that are configured to indicate whether or not new data is associated with a malignant or benign diagnosis.

In an example, the training data set **210B** may be analyzed to determine any dependencies, associations, and/or correlations between measured diagnosis and the benign/malignant statuses of the cases in the training data set **210B.** The identified correlations may have the form of a list of features that are present or absent for samples that are associated with different benign/malignant statuses. In an example, the training data set **210A** may be analyzed to determine one or more lists of features that have at least one feature in common with the training data set **210B.** These may be considered as features (or variables) in the machine learning context. The term "feature," as used herein, may refer to any characteristic of an item of data that may be used to determine whether the item of data falls within one or more specific categories. By way of example, the features described herein may comprise one or more diagnosis, pathologies, histologies, or the like or characteristics thereof.

In an example, a feature selection technique may comprise one or more feature selection rules. The one or more feature selection rules may comprise a feature occurrence rule. The feature occurrence rule may comprise determining which features in the training data set **210A** occur over a threshold number of times and identifying those features that satisfy the threshold as candidate features. For example, any features that appear greater than or equal to 2 times in the training data set **210A** may be considered as candidate features. Any features appearing less than 2 times may be excluded from consideration.

In an example, the one or more feature selection rules may comprise a significance rule. The significance rule may comprise determining, from the baseline data in the training data set **210B,** benign diagnosis data and malignant diagnosis data. As the baseline data in the training data set **210B** are labeled as benign or malignant, the labels may be used to determine the benign diagnosis data and malignant diagnosis data.

In an example, one or more candidate features may be selected according to a wrapper method. A wrapper method may be configured to use a subset of features and train a machine learning model using the subset of features. Based on the inferences that drawn from a previous model, features may be added and/or deleted from the subset. Wrapper methods include, for example, forward feature selection, backward feature elimination, recursive feature elimination, combinations thereof, and the like. Forward feature selection is an iterative method that begins with no feature in the machine learning model. In each iteration, the feature which best improves the model is added until an addition of a new variable does not improve the performance of the machine learning model. In an example, backward elimination may be used to identify one or more candidate features. Backward elimination is an iterative method that begins with all features in the machine learning model. In each iteration, the least significant feature is removed until no improvement is observed on removal of features. In an example, recursive feature elimination may be used to identify one or more candidate features. Recursive feature elimination is a greedy optimization algorithm which aims to find the best performing feature subset. Recursive feature elimination repeatedly creates models and keeps aside the best or the worst performing feature at each iteration. Recursive feature elimination constructs the next model with the features remaining until all the features are exhausted. Recursive feature elimination then ranks the features based on the order of their elimination.

In an example, one or more candidate features may be selected according to an embedded method. Embedded methods combine the qualities of filter and wrapper methods. Embedded methods include, for example, Least Absolute Shrinkage and Selection Operator (LASSO) and ridge regression which implement penalization functions to reduce overfitting. For example, LASSO regression performs L1 regularization which adds a penalty equivalent to absolute value of the magnitude of coefficients and ridge regression performs L2 regularization which adds a penalty equivalent to square of the magnitude of coefficients.

After training module **220** has generated a feature set(s), the training module **220** may generate a machine learning-based classification model **240** based on the feature set(s). Machine learning-based classification model, may refer to a complex mathematical model for data classification that is generated using machine-learning techniques. In one example, this machine learning-based classifier may include a map of support vectors that represent boundary features. By way of example, boundary features may be selected from, and/or represent the highest-ranked features in, a feature set.

In an example, the training module **220** may use the feature sets extracted from the training data set **210A** and/or the training data set **210B** to build a machine learning-based classification model **240A**-240N for each classification category (e.g., malignant, benign). In some examples, the machine learning-based classification models **240A-**240N may be combined into a single machine learning-based classification model **240.** Similarly, the machine learning-based classifier **230** may represent a single classifier containing a single or a plurality of machine learning-based classification models **240** and/or multiple classifiers containing a single or a plurality of machine learning-based classification models **240.**

The extracted features may be combined in a classification model trained using a machine learning approach such as discriminant analysis; decision tree; a nearest neighbor (NN) algorithm (e.g., k-NN models, replicator NN models, etc.); statistical algorithm (e.g., Bayesian networks, etc.); clustering algorithm (e.g., k-means, mean-shift, etc.); neural networks (e.g., reservoir networks, artificial neural networks, etc.); support vector machines (SVMs); logistic regression algorithms; linear regression algorithms; Markov models or chains; principal component analysis (PCA) (e.g., for linear models); multi-layer perceptron (MLP) ANNs (e.g., for non-linear models); replicating reservoir networks (e.g., for non-linear models, typically for time series); random forest classification; a combination thereof and/or the like. The resulting machine learning-based classifier **230** may comprise a decision rule or a mapping that assigns a case to a class (benign/malignant).

The machine learning-based classifier **230** may be used to predict the benign/malignant statuses (e.g., dichotomous outcomes) of the image sections in the image data or multiclass predictions of tissues (e.g., neoplastic, non-neoplastic, sessile serrated polyp, sessile serrated adenoma, serrated lesion). In one example, the result for each image section includes a confidence level that corresponds to a likelihood or a probability that the corresponding image section belongs in the predicted benign/malignant status. The confidence level may be a value between zero and one, which represents a likelihood that the corresponding test sample belongs to a benign/malignant status. In one example, when there are two statuses (e.g., benign and malignant), the confidence level may correspond to a value p, which refers to a likelihood that a particular image section belongs to a first status. In this case, the value 1-p may refer to a likelihood that the particular image section belongs to a second status.

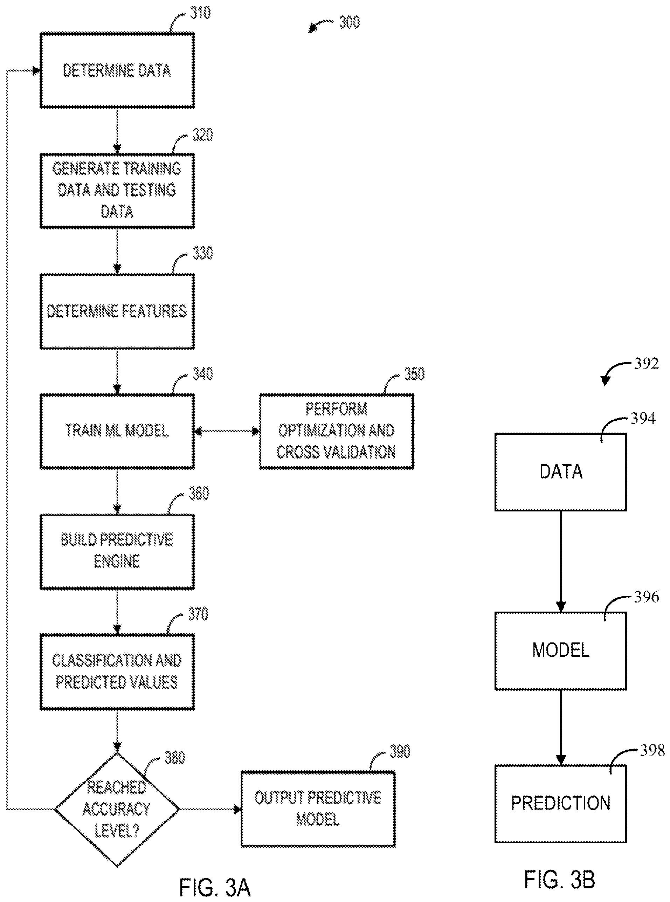

**FIG. 3A** is a flowchart illustrating an example training method **300** for generating the machine learning-based classifier **230** using the training module **220.** The training module **220** can implement supervised, unsupervised, and/or semi-supervised (e.g., reinforcement based) machine learning-based classification models **240.** The method **300** illustrated in **FIG. 3A** is an example of a supervised learning method; variations of this example of training method are discussed below, however, other training methods can be analogously implemented to train unsupervised and/or semi-supervised machine learning models.

The training method **300** may determine (e.g., access, receive, retrieve, etc.) first data (e.g., pathologies, histology etc...) of one or more studies and second data of one or more other studies at 310. The first data may contain one or more data sets, each data set associated with a particular study. Each study may include one or more diagnosis in common with the second data. First data may include segmentation ground truths along with input images, and second data may include classifications of regions of interest or pixel portions. Each study may involve different patient populations, although it is contemplated that some patient overlap may occur. The second data may contain one or more data sets, each data set associated with a particular study, different from those of the first data set. Each study may include one or more diagnosis in common with the first data. Each study may involve different patient populations, although it is contemplated that some patient overlap may occur. In an example, each data set may include a labeled list of diagnosis. In another example, each data set may comprise labeled baseline data. In another example, each data set may further include labeled in-treatment data.

The training method **300** may generate, at 320, a training data set and a testing data set. The training data set and the testing data set may be generated by randomly assigning labeled data of individual cases from the second data to either the training data set or the testing data set. In some implementations, the assignment of cases as training or test samples may not be completely random. In an example, only the labeled baseline data for a specific study may be used to generate the training data set and the testing data set. In an example, a majority of the labeled baseline data for the specific study may be used to generate the training data set. For example, 75% of the labeled baseline data for the specific study may be used to generate the training data set and 25% may be used to generate the testing data set. In another example, only the labeled in-treatment data for the specific study may be used to generate the training data set and the testing data set.

The training method **300** may determine (e.g., extract, select, etc.), at 330, one or more features that can be used by, for example, a classifier to differentiate among different classifications (e.g., malignant vs. benign). In an example, the training method **300** may determine a set of features from the first data. In another example, the training method **300** may determine a set of features from the second data. In another example, a set of features may be determined from data from a study different than the study associated with the labeled data of the training data set and the testing data set. In other words, data from the different study (e.g., the curated case agnostic data) may be used for feature determination, rather than for training a machine learning model. In an example, the training data set may be used in conjunction with the data from the different study to determine the one or more features. The data from the different study may be used to determine an initial set of features, which may be further reduced using the training data set.

The training method **300** may train one or more machine learning models using the one or more features at 340. In one example, the machine learning models may be trained using supervised learning. In another example, other machine learning techniques may be employed, including unsupervised learning and semi-supervised. The machine learning models trained at 340 may be selected based on different criteria depending on the problem to be solved and/or data available in the training data set. For example, machine learning classifiers can suffer from different degrees of bias. Accordingly, more than one machine learning models can be trained at 340, optimized, improved, and cross-validated at 350.

The training method **300** may select one or more machine learning models to build a predictive model at 360 (e.g., a machine learning classifier). The predictive model may be evaluated using the testing data set. The predictive model may analyze the testing data set and generate classification values and/or predicted values at 370. Classification and/or prediction values may be evaluated at 380 to determine whether such values have achieved a desired accuracy level. Performance of the predictive model may be evaluated in a number of ways based on a number of true positives, false positives, true negatives, and/or false negatives classifications of the plurality of data points indicated by the predictive model. For example, the false positives of the predictive model may refer to a number of times the predictive model incorrectly classified a feature as benign that was in reality a malignant. Conversely, the false negatives of the predictive model may refer to a number of times the machine learning model classified one or more features as malignant when, in fact, the feature was benign. True negatives and true positives may refer to a number of times the predictive model correctly classified one or more features as malignant or benign. Related to these measurements are the concepts of recall and precision. Generally, recall refers to a ratio of true positives to a sum of true positives and false negatives, which quantifies a sensitivity of the predictive model. Similarly, precision refers to a ratio of true positives a sum of true and false positives.

When such a desired accuracy level is reached, the training phase ends and the predictive model may be output at 390; when the desired accuracy level is not reached, however, then a subsequent iteration of the training method **300** may be performed.

In **FIG. 3B****,** an example application of a predictive model in accordance with one or more implementations of the present disclosure is illustrated. The process flow **392** may facilitate the prediction of an object, classification of an image, classification of a sub-image, or combinations thereof.

At step **394,** data can be determined. The data may comprise information related to tissue or a patient. As an example, the data may be an image or sub-image.

The data can be processed with a model at step **396,** and in step **398** a prediction can be made. The prediction may define a segmentation of pixels of an image. The prediction may define a classification of images or sub-images. The process flow **392** may be executed on a variety of hardware and software components as described herein including various network structures and learning methods.

**FIG. 4** shows a plurality of images **410, 420, 430, 440,** 450, and 460. Images **410, 420,** and 430, comprise image data comprising pictures of predicted objects. The predicted objects may comprise polyps. Images **440, 450,** and 460 comprise composite images comprising an object map overlaid over the predicted object. The object map, as described herein, may comprise a visual representation of data, pathologies, histologies, and the like associated with the predicted object. In an example, the object map may comprise regions or subregions. For example, 440 shows a composite image wherein the object map comprises two regions (green and yellow). The colors associated with a region may be indicative of feature and/or or confidence score. For example, a green color may be indicative of a high confidence that the region is benign while a yellow color may be indicative of a low confidence as to whether a region is benign or malignant. Likewise, as can be seen in image **450,** the predicted object may comprise a single region and/or a single color. For example, in image **450,** the object map is entirely red, which may indicate a high confidence that the region is malignant. Image **460** shows an example composite image wherein the object map comprises a plurality of colors associated with a plurality of regions of the predicted object.

**FIGS. 5A-5B** show several example visual indications or representations associated with a predicted object. The visual representation may comprise a plurality of scores. For example, the visual representation may comprise scores ranging from 1 to 5. The score may comprise confidence scores. Generating image data for the visual representation may comprise imaging an area of the body several times to determine a predicted object. The several images of the predicted object may vary with regard to their visual representation. The scores making up the visual representation may comprise color indicators (as seen in **FIG. 5B****).** As an example, the several visual representations may be stacked or overlaid as shown in **FIG. 5B****.** The visual representations may partially overlap or completely overlap. One value may represent a high confidence score while another value represents a low confidence score. Likewise, a color may be associated with a predicted diagnosis such as benign while another color may be associated with a different diagnosis such as malignant. As can be seen, visual representation **510, 520,** and **530** have been partially overlapped and the scores of each image section added. In **FIG 5B****,** the overlapping visual representation have generated an object map that has a high confidence of malignancy as seen by the red "5s."

**FIG. 6** shows an example method comprising receiving, at step **610,** image data, determining, at step **620,** an initial classification associated with an image section, determining, at step **630,** an additional initial classification associated with an additional image section, determining, at step **640** a score, determining, at step **650,** a visual indication and outputting, at step **660,** an object map. Such steps may include one or more steps from **FIG. 1****.**

Receiving image data at step **610** may comprise downloading, obtaining, or receiving the image data, or combinations thereof. The image may be obtained from various sources, including live feeds or streams, databases, live-video, real-time images, other image data and the like. The image data may be received from a machine learning classifier as described above. The image data may comprise at least one region. For instance, one region may comprise a feature, while another region comprises another feature. Further, the image may comprise sections. For example, the sections may comprise a pixel or a voxel or a plurality of pixels or voxels groups of pixels or voxels or the like. In an example, a section may comprise 32 pixels in one direction (e.g., the x direction), and 32 pixels in another direction (the y or z direction) or combinations thereof.

Determining an initial classification at step **620** may comprise making a determination as to a feature, diagnosis, pathology, histology or the like. For instance, determining an initial classification may comprise determining the image section comprises a feature, such as a polyp or a boundary or some characteristic, for example a surface characteristic. The initial classification may comprise a classification as to whether that image section is indicative of, for example, a section of a polyp being benign or malignant.

Determining an additional classification at **630** may comprise making a determination as to a feature, diagnosis, pathology, histology or the like. For instance, determining an initial classification may comprise determining the image section comprises a feature, such as a polyp or a boundary or some characteristic, for example a surface characteristic. The initial classification may comprise a classification as to whether that image section is indicative of, for example, a section of a polyp being benign or malignant.

In an example, the initial classification of step **620** may use the semantic segmentation model to determine boundaries of growths within the image. For example, the semantic segmentation may classify individual pixels of the image as those related to growths and those related to ordinary tissue. The additional classification of step **630** may use the trained region of interest model to classify the images as neoplastic and non-neoplastic. For example, smaller regions of interest having pixels within the boundary may be classified as neoplastic and non-neoplastic. As another example, the additional classification of step **630** may classify every pixel within the boundary or a scaled version of the image associated with the boundary as neoplastic or non-neoplastic, providing a coarse indication of abnormal growth.

Determining a score at step **640** may comprise determining a confidence score based on the initial classification, any additional classifications, or combinations thereof. For instance, the confidence score may be associated with an initial classification of malignancy. The confidence score may be associated with the initial classification of malignancy, a first additional classification of malignancy, a second additional classification of malignancy, other additional classifications, or combinations thereof. In such a case, the confidence score may adjusted lower than the initial classification of malignancy in a scenario where the initial classification is malignancy and a first additional classification is benign. In an example, the score of step **640** may be determined based on part of the classification process. For instance, the score may be a confidence score related to the classification of the region of interest as neoplastic against the classification of the region of interest as non-neoplastic.

Determining a visual indication at step **650** may comprise associating a plurality of image sections with initial classifications. Determining a visual representation may comprise associating additional image sections with additional classifications. The image sections and additional image sections may be overlaid so as to develop an object map which may comprise at least one visual indication. Determining a visual indication at **650** may comprise associating the score with the initial image section and/or the additional image section. In an example, step **650** may comprise adding, augmenting, or otherwise inserting a visual representation of the classification as a color, symbol, pattern, or otherwise. For instance, the classification may be related to coloration of the particular region of interest or portion of the pixels classified. As shown in **FIG. 4****,** a hue of the region of interest or portion may be changed. As an example, regions of interest classified as neoplastic may be shaded red. As another example, regions of interest classified as non-neoplastic may be shaded green. The underlying structure may be left intact such that a viewer can see which area of the original image is identified as neoplastic.

Outputting an object map at step **660** may comprise sending the object map to a display device. Outputting the object map may comprise displaying the object map on the display device.

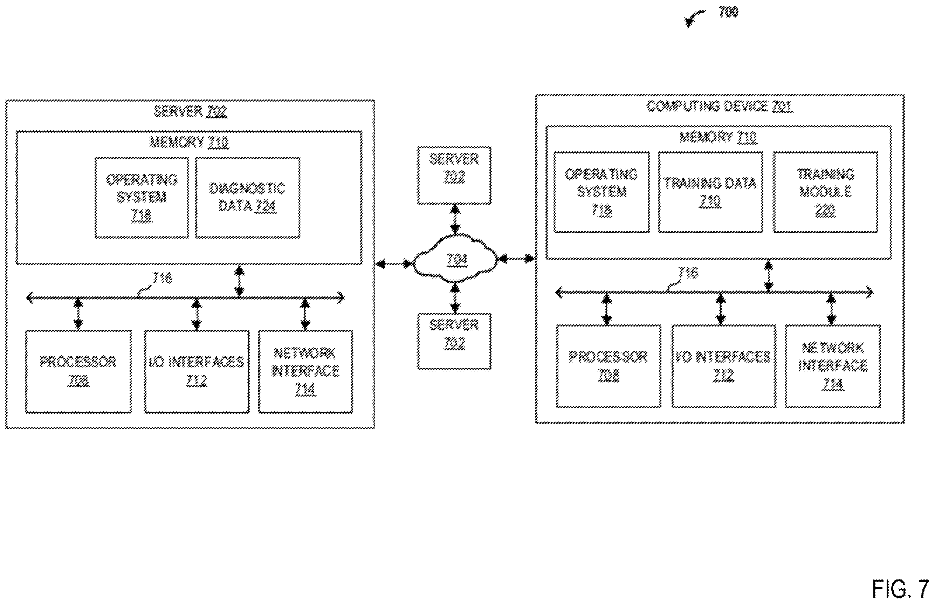

**FIG. 7** is a block diagram depicting an environment **700** comprising nonlimiting examples of a computing device **701** and a server **702** connected through a network **704.** In an aspect, some or all steps of any described method may be performed on a computing device as described herein. The computing device **701** can comprise one or multiple computers configured to store one or more of the training module **220,** training data **210** (e.g., labeled baseline data, labeled in-treatment data, and/or curated case agnostic data), and the like. The server **702** can comprise one or multiple computers configured to store data **724** (e.g., curated case agnostic data). Multiple servers **702** can communicate with the computing device **701** via the through the network **704.**

The computing device **701** and the server **702** can be a digital computer that, in terms of hardware architecture, generally includes a processor **708,** memory system **710,** input/output (I/O) interfaces **712,** and network interfaces **714.** These components **(708, 710, 712,** and 714) are communicatively coupled via a local interface **716.** The local interface **716** can be, for example, but not limited to, one or more buses or other wired or wireless connections, as is known in the art. The local interface **716** can have additional elements, which are omitted for simplicity, such as controllers, buffers (caches), drivers, repeaters, and receivers, to enable communications. Further, the local interface may include address, control, and/or data connections to enable appropriate communications among the aforementioned components.

The processor **708** can be a hardware device for executing software, particularly that stored in memory system **710.** The processor **708** can be any custom made or commercially available processor, a central processing unit (CPU), an auxiliary processor among several processors associated with the computing device **701** and the server **702,** a semiconductor-based microprocessor (in the form of a microchip or chip set), or generally any device for executing software instructions. When the computing device **701** and/or the server **702** is in operation, the processor **708** can be configured to execute software stored within the memory system **710,** to communicate data to and from the memory system **710,** and to generally control operations of the computing device **701** and the server **702** pursuant to the software.

The I/O interfaces **712** can be used to receive user input from, and/or for providing system output to, one or more devices or components. User input can be provided via, for example, a keyboard and/or a mouse. System output can be provided via a display device and a printer (not shown). I/O interfaces **712** can include, for example, a serial port, a parallel port, a Small Computer System Interface (SCSI), an infrared (IR) interface, a radio frequency (RF) interface, and/or a universal serial bus (USB) interface.

The network interface **714** can be used to transmit and receive from the computing device **701** and/or the server **702** on the network **704.** The network interface **714** may include, for example, a 10BaseT Ethernet Adaptor, a 100BaseT Ethernet Adaptor, a LAN PHY Ethernet Adaptor, a Token Ring Adaptor, a wireless network adapter (e.g., WiFi, cellular, satellite), or any other suitable network interface device. The network interface **714** may include address, control, and/or data connections to enable appropriate communications on the network **704.**

The memory system **710** can include any one or combination of volatile memory elements (e.g., random access memory (RAM, such as DRAM, SRAM, SDRAM, etc.)) and nonvolatile memory elements (e.g., ROM, hard drive, tape, CDROM, DVDROM, etc.) that are non-transitory. Moreover, the memory system **710** may incorporate electronic, magnetic, optical, and/or other types of storage media. Note that the memory system **710** can have a distributed architecture, where various components are situated remote from one another, but can be accessed by the processor **708.**

The software in memory system **710** may include one or more software programs, each of which comprises an ordered listing of executable instructions for implementing logical functions. In the example of **FIG. 7****,** the software in the memory system **710** of the computing device **701** can comprise the training module **220** (or subcomponents thereof), the training data **210B,** and a suitable operating system (O/S) 718. In the example of **FIG. 7****,** the software in the memory system **710** of the server **702** can comprise, the data **724,** and a suitable operating system (O/S) 718. The operating system **718** essentially controls the execution of other computer programs and provides scheduling, input-output control, file and data management, memory management, and communication control and related services. One or more of the components may be used in an endoscope.

For purposes of illustration, application programs and other executable program components such as the operating system **718** are illustrated herein as discrete blocks, although it is recognized that such programs and components can reside at various times in different storage components of the computing device **701** and/or the server **702.** An implementation of the training module **220** can be stored on or transmitted across some form of computer-readable media. Any of the disclosed methods can be performed by computer-readable instructions embodied on computer-readable media. Computer-readable media can be any available media that can be accessed by a computer. By way of example and not meant to be limiting, computer readable media can comprise "computer storage media" and "communications media." "Computer storage media" can comprise non-volatile, removable and non-removable media implemented in any methods or technology for storage of information such as computer readable instructions, data structures, program modules, or other data. Example computer storage media can comprise RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computer.

Referring to **FIG. 8****,** a decolorized version of **FIG. 4** is illustrated in accordance with one or more implementations of the present disclosure. **FIG. 8** is intended to clarify portions of the previously disclosed subject matter without adding additional matter. As shown in Figs. 4 and 8, original endoscopic images **410, 420, 430** are taken during a colonoscopy or another operation. The original endoscopic images **410, 420, 430** present ordinary tissue along with unordinary tissues **812, 822, 832.** As shown in composite images **440, 450, 460** indicia is added to the original endoscopic images **410, 420, 430** to highlight the classification of the image and the confidence score of the classification. For instance, original endoscopic image **410** include a single growth, object, abnormal, or unordinary tissue **812.**

A boundary **842** is defined by the classification or segmentation of the pixels of the image to identify the unordinary tissue **812.** The original endoscopic images **420, 430** are also associated with respective boundaries **852, 862** defined by the classification or segmentation of the pixels of the image. Indicia **870, 872, 874, 876** may be imparted on the portion of the image within the boundary using classification of regions of interest or portions of the image with the classification model, detecting neoplastic and non-neoplastic tissue through image classification.

The indicia **870, 872, 874, 876** may change the hue of the original endoscopic image to allow a gastroenterologist to see the underlying tissue and its classification. As shown, a map of different indicia indicates the classification and the confidence score. For example, indicia **870** may be a red overlay defining bounded areas of neoplastic tissue within the respective boundaries **842, 852, 862.** Indicia **872** may be a green overlay defining bounded areas of neoplastic tissue within the boundary **842, 852, 862.** Indicia **874, 876** may be yellow augment to the red and green overlays. For instance, a low confidence score neoplastic area may be represented as a yellowish red, shown as indicia **876.** As another example, a low confidence score non-neoplastic area may be represented as a yellowish green, shown as indicia **874.** Various types of indicia may be used. Overlays and symbols may be used to indicate respective areas. A different hue or coloration scheme may be used. As an example, the colorblind may prefer yellow and blue as opposed to red and green.

In **FIG. 9A****,** a training method for semantic segmentation or pixel classification in accordance with one or more implementations of the present disclosure is shown as method **900.** **FIG. 9A** is intended to clarify portions of the previously disclosed subject matter without adding additional matter. A segmentation model **908** may be trained to identify boundaries within an image **410.** As an example, image **410** is processed by the semantic segmentation model **908** for a prediction of the correct segmentation. The semantic segmentation model **908** may be trained to output a segmented image **910** with segmentations **914, 916** classified to according to the annotated training image **902.** The annotated training image **902** is defined with the ground truth semantic segmentations **904, 906.** The ground truth semantic segmentations may provide a labeled boundary **907** explicitly or implicitly by virtue of border of the ground truth semantic segmentations **904, 906.** As an example, the semantic segmentation model **908** may be trained against the annotated training image **902,** comparing the classification of pixels of the segmented image **910** to the annotated training image **902.** After segmentations are defined in the segmented image **910,** the boundary **912** may be explicitly or implicitly determined where the segmentations **914, 916** abut. The semantic segmentation model **908** may then be used to segment other images once trained. The semantic segmentation model **908** may be an encoder-decoder network that uses atrous convolutions. The convolutions may further include residual learning of convolutional layers.

In **FIG. 9B****,** a training method for a region of interest or pixel portion classifier in accordance with one or more implementations of the present disclosure is shown as method **950.** **FIG. 9B** is intended to clarify portions of the previously disclosed subject matter without adding additional matter. The **ROI** classifier **960** may be trained with regions of interest or pixel portions of the image **410** and other images. Training data **210B** selections may be made from within the boundary **912** identified with the semantic segmentation model **908.** For example, training images **952, 954, 956,** which are also sub-images of the image **410,** and may all be selected from within the boundary **912** and annotated as neoplastic or non-neoplastic, benign or malignant, or various other categorizations (e.g., predicted disease state). The training images **952, 954, 956** are used to train the ROI classifier **960,** adjusting the model based on the loss associated with classification **962.** The model may be a residual network of convolutions. As an example, shown throughout this disclosure and **FIG. 9B****,** regions or portions of images may be compiled and identified as neoplastic or non-neoplastic. The region-of-interest classifier **960** may be trained on these sub-images and classifier identifications from training data set **210B** to properly predict the nature of the region, portion, or sub-image. The training data set **210B** may only include images that are associated or within boundaries defined by the segmentation model. The training data set **210B** may include thousands of classified images (e.g., 65,000). In an example, determining a given probability or a probability distribution may comprise calculating the probability distribution for a given feature based on the values of the features and prior medical knowledge, wherein the prior medical knowledge comprises one or more parameter values, generating a probability map by using Bayes' Law to combine the probability distributions.

In **FIG. 10****,** a portion of a classification method for classifying a region of interest or pixel portion with a trained classification model in accordance with one or more implementations of the present disclosure is shown as method **1000.** **FIG. 10** is intended to clarify portions of the previously disclosed subject matter without adding additional matter. In step **1004,** the image **412** or any other image is received from a repository or medical instrument. The image may include segmentation data indicative of a boundary **1002.** As discussed, assignments for each pixel of the image **412** may be determined through the semantic segmentation model **908** or another implement. Maximal dimensions **1012** of the boundary **1002** may be determined to resize the received image **412** to resized image **1006.** The resized image **1006** may be defined by predefined dimensions based on the maximal dimensions **1012.** As an example, the dimensions **1008, 1010** may be 384 pixels by 384 pixels. As another, the dimensions **1008, 1010** may be 512 pixels by 256 pixels or 256 pixels by 512 pixels. Resizing may be based on the maximal length and the maximal width, maximal dimensions **1012,** of the boundary **1002.** If the maximal length and the maximal width, maximal dimensions **1012,** are similar or within a specific ratio (e.g., 3:4), the boundary **1002** is considered squarer than oblong and the 384 pixels by 384 pixels square is used for resizing. If the maximal length and the maximal width , maximal dimensions **1012,** are dissimilar or outside of a specific ratio (e.g., 3:4), the boundary **1002** is considered more oblong than square and the 512 pixels by 256 pixels rectangle, or 256 pixels by 512 pixels) is used for resizing. In such a way, the underlying features of the images **412** are maintained without altering the processing of the image.

Resizing the image **412** into resized image **1006** may provide a more constant or stable processing timeframe, which can ensure presentation to an operator at constant or similar intervals. For instance, processing images with one million pixels may take longer than processing images with one thousand pixels, allowing this process to provide real-time or near real-time results (e.g., one frame per second, ten frames per second, 30 frames per second) regardless of the input image and without loss in quality.

The resizing process may include cropping the image **412** according to the boundary **1002** and then resizing the image to the configured size. As an example, the image **412** may be cropped into a rectangular image around the boundary **1002** based on the maximal pixel locations and the minimal pixel locations. Padding or extra portions of the image **410** may be include (e.g., maximal locations plus five and minimal locations minus five). As such, the resulting rectangle is based on the size of the segmented object. The processing time for classifying regions of interest or pixel portions within the segmented object may be constant for all objects detected during the procedure by resizing (e.g., upscaling or downscaling) the rectangle of pixels associated with the boundary **1002.** As an example, during upscaling pixel values may be expanded or duplicated, and during downscaling pixel values may be averaged or otherwise reduced to maintain the same content. As such, the processing time required is held constant or predictable to ensure real-time or same-time availability of classification information at the instrument or display.

In step **1018,** a region of interest or pixel portion may be selected. As an example, the entire set of pixels **1014** from resized image **1006** may be selected for classification. The classification and resulting confidence score may be used to adjust indicia provided. As an example, if resized image **1006** is neoplastic the hue of all pixels may be shaded red. The strength of the shading may be based on the confidence score and a limit may be used to ensure that the entire image classification is not overused.

As another example, a region of interest or pixel portion **1016** may be selected that includes a portion of the set of pixels from resized image **1006.** For instance, the pixel portion **1016** may be 64-pixels by 64-pixels. Selections may have at least one pixel within the boundary **1002.** After classification, indicia related to the pixel portion **1016** outside of the boundary **1002** may be removed or zeroed.

In step **1020,** the pixel portion **1016** may be classified using the ROI classifier **960,** as trained. The classifier may output a classification **1022** and a confidence score **1024.**

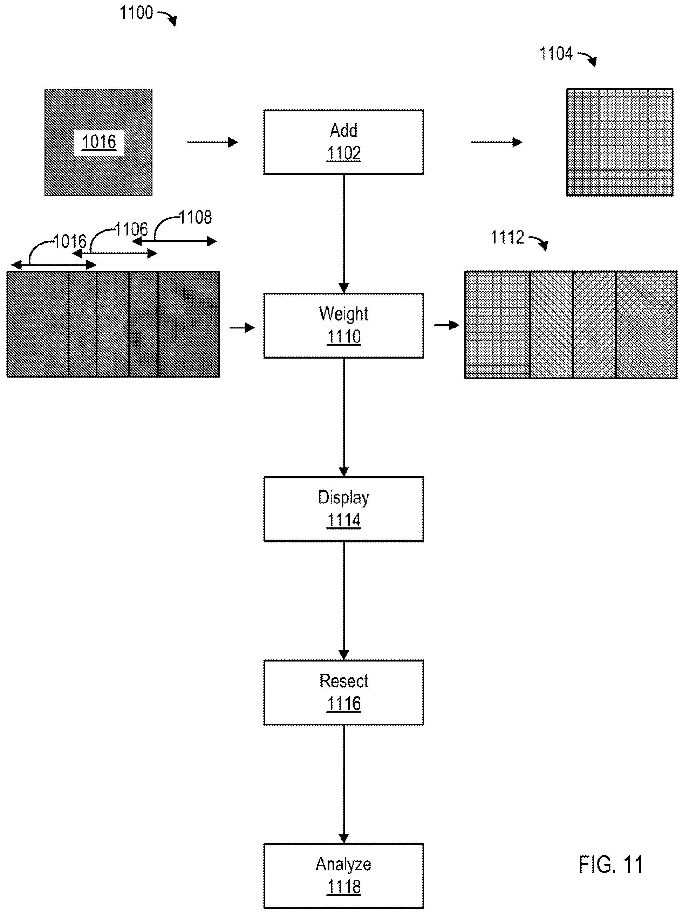

Referring to **FIG. 11****,** a portion of a treatment for tissue in accordance with one or more implementations of the present disclosure is shown in method **1100.** FIG. 11 is intended to clarify portions of the previously disclosed subject matter without adding additional matter. In step **1102,** indicia **870, 872, 874, 876** is added to the region of interest or pixel portion **1016.** The indicia **870, 872, 874, 876** may be various implements. As an example, the indicia **870, 872, 874, 876** may alter a hue of the pixel portion **1016.** In such a case, the indicia **870, 872, 874, 876** may be added by multiplying red, blue, and green ("RGB") color values of each pixel of the pixel portion **1016** with a modifier or adding to the color values. Other color components may be used. (e.g. "YUV"). As an example, the pixel may have a red value between zero and 255 (e.g., five). To add the indicia, the red value of the pixel may be multiplied by ten or have 128 added to it. As such, the hue will be changed as shown in **FIG. 5A** and **FIG. 5B****.**

In step **1110,** weighting is applied from adjacent pixel portions **1016, 1106, 1108** to adjust the indicia of respective pixel portions **1016, 1106, 1108.** As an example, the classification of pixel portion **1106** is used to adjust the pixel portion **1016.** The ROI classifier may classify pixel portion **1106** as neoplastic with low confidence while classifying pixel portion **1106** as neoplastic with high confidence. As such, the pixels corresponding to the non-overlapped portion of pixel portion **1016** may be red. The overlapped portion between pixel portion **1016** and pixel portion **1106** may be represented as yellowish red. The change in hue may be obtained by increasing the green component of the pixels within both pixel portion **1016** and pixel portion **1106.** As such, the non-overlapped portion of 1016 may be a stronger red or indicia with green as zero and red near 255, the overlapped portion a moderate yellowish red with red near 255 and green near 100, and the non-overlapped portion of 1106 may be a mild yellowish red with red near 255 and green near 200. Such an application serves to blend the neighboring pixel portions **1016, 1106, 1108,** as also shown in **FIG. 5B****.** Adjacent pixel portions **1016, 1106, 1108** may be overlapped a certain percentage and although shown in a row, adjacent rows of the pixel portions **1016, 1106, 1108** may overlap to provide a two-dimensional or three-dimensional blending to provide result image **1112.**

In step **1114,** the result image **1112** is displayed on the instrument or associated display. The result image **1112** may be one of the composite images **440, 450, 460** and can be displayed alongside the original image **412** for comparison or on its own because the indicia **870, 872, 874, 876** maintains the underlying image while providing information to an endoscopist. The result image **1112** may be displayed in real-time based on the technical improvements described. As such, resection may be performed by an endoscopist to remove the growth based on the result image **1112** in step **1116.** As an example, an endoscopist may be trained to resect tissue based on the coloration and stratification of the result image. An endoscopist may be trained to resect only portions of the tissue with a red hue or yellowish-red hue. As another example, an endoscopist may be trained to resect tissue outlined by the the boundary **1002** when the internal map is substantially indicative of neoplastic tissue (e.g., substantially red or yellowish-red). As another example, the resection of tissue may be performed based on a size of the boundary **1002** and the classification of the regions of interest or pixel portions. For instance, boundaries **1002** that represent growths or polyps less than five millimeters may be resected based on the regions of interest being substantially red or yellowish-red, and boundaries **1002** that represent growths or polyps greater than five millimeters may be resected regardless of the indicia **870, 872, 874, 876** or when the indicia **870, 872, 874, 876** is not entirely green. Various decision hierarchies can be devised to ensure that the proper resection is performed based on the indicia **870, 872, 874, 876** and the shapes or sizes of the boundary **1002.**

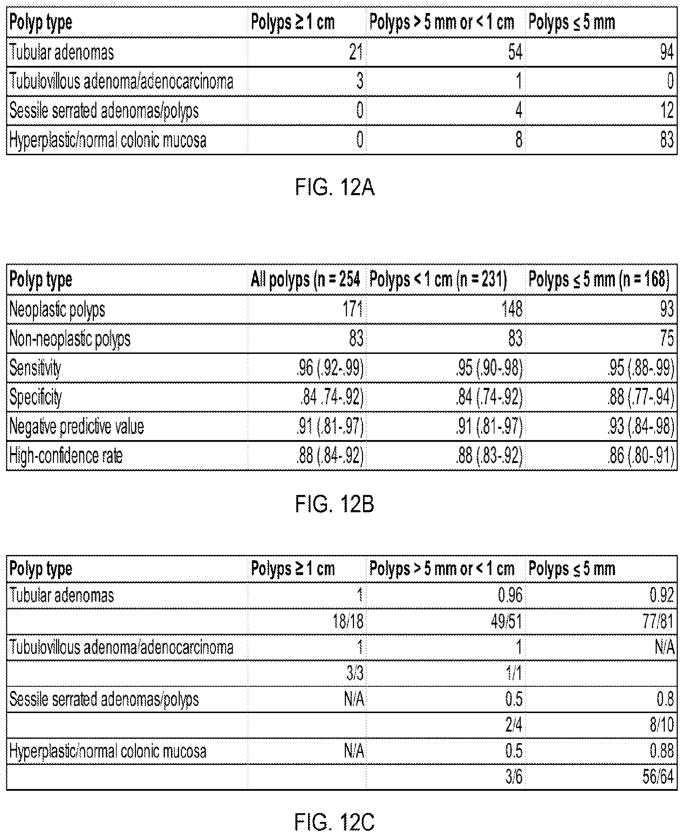

In **FIG. 12A****,** statistical data of polyp pathology in accordance with one or more implementations of the present disclosure is shown. For example, prevalence of tubular adenomas, tubulovillous adenomas, sessile serrated adenomas and hyperplastic colonic mucosa is disclosed. Indeed, provided are methods and systems for reducing errors in the display of medically imaged tissue data and the treatments thereof. As an example, the provided techniques open the box of the previously unknown, and in one example, provide both an outline of identified polyp boundaries within which histology assessments are made and a map of spatially localized histology predictions in subregions within the detected boundary. Indeed, in this possible enhancement of the many provided herein, an intuitive, color-enriched, augmented visualization of the model's predicted histology over the polyp surface, providing an enhanced level of transparency and interpretability.

In an example, a sensitivity of 0.96, specificity of 0.84, negative predictive value (NPV) of 0.91, and high-confidence rate (HCR) of 0.88, distinguishing 171 neoplastic polyps from 83 non-neoplastic polyps of all sizes, or better, could be achieved. Among 93 neoplastic and 75 non-neoplastic polyps <5 mm, the model achieved a sensitivity of 0.95, specificity of 0.84, NPV of 0.91, and HCR of 0.86.

**FIG. 12B** illustrates statistical data of computer-aided treatment in accordance with one or more implementations of the present disclosure. For example, performance results of computer-aided treatment regarding neoplastic polyps non-neoplastic polyps is shown.

**FIG. 12C** illustrates statistical data of confidence in computer-aided treatement in accordance with one or more implementations of the present disclosure. For example, performance results of computer-aided treatment regarding various polyp sizes is shown.

As one or more examples, a method for treating tissue of a patient may include capturing an image of the tissue. The captured image having pixels arranged to provide a visual representation of the tissue. The method may include segmenting the pixels to designate a boundary. The method may include selecting a first pixel portion of the pixels based on a first tissue portion of the tissue. The first pixel portion may have a pixel of the pixels within the boundary.

The method may include classifying the first pixel portion for resection with a confidence score. The confidence score may be indicative of a negative positive value greater than 80% for classification of neoplastic and non-neoplastic tissue. The method may include resecting the first tissue portion according to the confidence score.

The method may include adding indicia to the first pixel portion and displaying the first pixel portion with the indicia. The indicia may change a hue of the pixel portion. The pixel may be a first pixel and the indicia may be weighted according to the first pixel portion and a second pixel portion. The second pixel portion may be based on the first tissue portion. The second pixel portion may have a second pixel of the pixels within the boundary.

Pixels of the first pixel portion may comprise or essentially consist of every pixel within the boundary and the pixels of the second pixel portion may be a 64-pixel-by-64-pixel contiguous block of the pixels. Pixels of the first pixel portion may be a first 64-pixel-by-64-pixel contiguous block of the pixels and the pixels of the second pixel portion may be a second 64-pixel-by-64-pixel contiguous block of the pixels. A fourth of the pixels of the first pixel portion may be equal to pixels of the second pixel portion.

The method may include selecting a third pixel portion of the pixels based on the first tissue portion. The third pixel portion may have a third pixel of the pixels within the boundary and the fourth of the pixels of the first pixel portion. The method may include changing a color composition of the pixels of the first pixel portion within the boundary. The method may include maintaining the color composition of the pixels of the of the first pixel portion outside the boundary.

The method may include masking the pixels of the first pixel portion outside the boundary such that only the pixels of the first pixel portion within the boundary are used for classification.

The method may include maintaining the color composition of the pixels of the of the first pixel portion based on a percentage of the pixels of the first pixel portion within the boundary.

The method may include classifying a second pixel portion for abandonment. The classifying may include adding first indicia to the first pixel portion and adding second indicia different from the first indicia to the second pixel portion.

The method may include discarding the first tissue portion. The may include analyzing the first tissue portion for cancer.

The method may include extracting features of the pixels according to a deep-learning network. The method may include decoding the extracted features to designate the boundary that categorizes the pixels to represent a growth within the tissue,

Segmenting the pixels may be based on a trained model. The model may be trained according to previously captured images annotated with the segments. The annotations may identify pixels associated with the segments and categorizing the pixels.

Classifying the portion may be based on a trained model of classified images. The classified images may be classified as benign or cancerous, neoplastic or non-neoplastic.

The first pixel portion is a 64-pixel-by-64-pixel square of the pixels. The captured image may be resized according to the boundary. The resizing may be an upsample of the pixels to a defined quantity such that computation of the classifying is substantially similar for iterations of the method. The resizing may be downsample of the pixels to a defined quantity such that computation of the classifying is substantially similar for iterations of the method.

The defined quantity may be 384 pixels by 384 pixels for a boundary that is squarer than oblong. The defined quantity may be 512 pixels by 256 pixels for a boundary that is more oblong than square.

The method may include receiving, from a machine learning classifier, object classification data associated with image data. The object classification data may comprise an indication of a predicted object, an indication of one or more regions of the predicted object, and one or more scores associated with the indication of the one or more regions of the predicted object. The method may include determining, based on the indication of the predicted object, data indicative of a boundary of the predicted object. The method may include determining, based on the indication of the one or more regions of the predicted object and the one or more scores associated with the indication of the one or more regions of the predicted object, a visual representation associated with each of the one or more regions of the predicted object.

The method may include determining, based on the data indicative of the boundary of the predicted object, and the visual representation associated with each of the one or more regions of the predicted object, an object map. The method may include outputting, based on the object map and the image data, a composite image.

The object classification data may include a score associated with the indication of the predicted object. The indication of the predicted object may include at least one of an object class or object coordinates. The one or more scores associated with the indication of the one or more regions of the predicted object may include a confidence score. The image data may include at least one of a digital image or a video.

The method may include outputting the composite image on a display device. The method may include determining, based on the one or more scores associated with the indication of the one or more regions of the predicted object, a color associated with the one or more scores associated with the indication of the one or more regions of the predicted object. The determining the visual representation associated with each of the one or more regions of the predicted object may include assigning the color to the visual representation associated with each of the one or more regions of the predicted object.

The method may include determining, based on the object map, an object prediction. The method may include outputting, with the composite image, the object prediction.

A method may include receiving, from a machine learning classifier, image data, wherein the image data comprises at least one region and a plurality of image sections, and wherein the at least one region comprises at least one subregion. The method may include determining, based on at least one image section of the plurality of image sections, an initial classification associated with the at least one image section. The method may include determining, based on at least one additional image section of the plurality of image sections, an additional classification associated with the at least one additional image section. The method may include determining, based on the initial classification and the additional classification, a score, wherein the score is associated with the at least one subregion. The method may include determining, based on the score, a visual indication. The method may include outputting, based on the visual indication, an object map.

The method may include determining the score comprises overlaying the at least one image section and the at least one additional image section and averaging the initial classification and the additional classification. The method may include at least one of an object class, object coordinates, pathology. The image data may include at least one of a digital image or video. Outputting the composite image may include outputting the composite image on a display device. The plurality of image sections may include pixels.

A system may include a computing device. The computing device may be a processor or group of processors. Instructions may be stored on digital storage. The instructions may be operable upon execution by the processor to receive, from a machine learning classifier, object classification data associated with image data. The object classification data may include an indication of a predicted object, an indication of one or more regions of the predicted object, and one or more scores associated with the indication of the one or more regions of the predicted object. The instructions may be operable upon execution by the processor to determine, based on the indication of the predicted object, data indicative of a boundary of the predicted object. The instructions may be operable upon execution by the processor to determine, based on the indication of the one or more regions of the predicted object and the one or more scores associated with the indication of the one or more regions of the predicted object, a visual representation associated with each of the one or more regions of the predicted object. The instructions may be operable upon execution by the processor to determine, based on the data indicative of the boundary of the predicted object, and the visual representation associated with each of the one or more regions of the predicted object, an object map. The system may include a display device. The display device may be configured to output, based on the object map and the image data, a composite image.

The computing device may be configured to receive a score associated with the indication of the predicted object. The indication of the predicted object may include at least one of an object class or object coordinates. The one or more scores associated with the indication of the one or more regions of the predicted object may include a confidence score. The computing device may be configured to output the composite image on the display device. The instructions may be operable upon execution by the processor to determine, based on the one or more scores associated with the indication of the one or more regions of the predicted object, a color associated with the one or more scores associated with the indication of the one or more regions of the predicted object. The visual representation associated with each of the one or more regions of the predicted object may include assigning the color to the visual representation associated with each of the one or more regions of the predicted object.

An apparatus may include one or more processors. The apparatus may include memory storing processor executable instructions that, when executed by the one or more processors, may cause the apparatus to receive, from a machine learning classifier, object classification data associated with image data. The object classification data may include an indication of a predicted object, an indication of one or more regions of the predicted object, and one or more scores associated with the indication of the one or more regions of the predicted object. The instructions may cause the apparatus to determine, based on the indication of the predicted object, data indicative of a boundary of the predicted object. The instructions may cause the apparatus to determine, based on the indication of the one or more regions of the predicted object and the one or more scores associated with the indication of the one or more regions of the predicted object, a visual representation associated with each of the one or more regions of the predicted object. The instructions may cause the apparatus to determine, based on the data indicative of the boundary of the predicted object, and the visual representation associated with each of the one or more regions of the predicted object, an object map. The instructions may cause the apparatus to output, based on the object map and the image data, a composite image.

The method steps recited throughout this disclosure may be combined, omitted, rearranged, or otherwise reorganized with any of the figures presented herein and are not intend to be limited to the four corners of each sheet presented. Any one or more of the method steps disclosed herein may be performed in whole or in part by one or more computers or stored as executable instructions on a computer-readable medium.

It is understood that when combinations, subsets, interactions, groups, etc. of components are described that, while specific reference of each various individual and collective combinations and permutations of these may not be explicitly described, each is specifically contemplated and described herein. This applies to all parts of this application including, but not limited to, steps in described methods. Thus, if there are a variety of additional steps that may be performed it is understood that each of these additional steps may be performed with any specific configuration or combination of configurations of the described methods.

As will be appreciated by one skilled in the art, hardware, software, or a combination of software and hardware may be implemented. Furthermore, a computer program product on a computer-readable storage medium (e.g., non-transitory) having processor-executable instructions (e.g., computer software) embodied in the storage medium. Any suitable computer-readable storage medium may be utilized including hard disks, CD-ROMs, optical storage devices, magnetic storage devices, memresistors, Non-Volatile Random Access Memory (NVRAM), flash memory, or a combination thereof.

Throughout this application reference is made to block diagrams and flowcharts. It will be understood that each block of the block diagrams and flowcharts, and combinations of blocks in the block diagrams and flowcharts, respectively, may be implemented by processor-executable instructions. These processor-executable instructions may be loaded onto a special purpose computer or other programmable data processing apparatus to produce a machine.

These processor-executable instructions may also be stored in a computer-readable memory that may direct a computer or other programmable data processing apparatus to operate in a particular manner, such that the processor-executable instructions stored in the computer-readable memory produce an article of manufacture including processor-executable instructions for implementing the operation specified. The processor-executable instructions may also be loaded onto a computer or other programmable data processing apparatus to cause a series of operational steps to be performed on the computer or other programmable apparatus to produce a computer-implemented process.

The specialized steps discussed herein do not seek to forestall of sets of instructions executable by a computer to obtain the results specified herein. Indeed, the specific steps and methods described herein are just a few possible solutions to the treatment of tissue within a patient. The present invention has been described with a certain degree of particularity but it should be understood that various modifications and alterations may be made without departing from the scope or spirit of the invention as defined by the following claims. The provisional application which this application claims benefit is incorporated by reference in its entirety, and any original disclosure of specification, figures, and claims are expressly maintained herein.

While the methods and systems have been described in connection with preferred embodiments and specific examples, it is not intended that the scope be limited to the particular embodiments set forth, as the embodiments herein are intended in all respects to be illustrative rather than restrictive.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; the number or type of embodiments described in the specification.

It will be apparent to those skilled in the art that various modifications and variations can be made without departing from the scope of protection defined by the claims. Other embodiments will be apparent to those skilled in the art from consideration of the specification and practice disclosed herein. It is intended that the specification and examples be considered as exemplary only, with a true scope being indicated by the following claims.

## Claims

1. A computer implemented method comprising:
determining, based on a first classifier, an object in image data;
determining, based on the object, a boundary of the object;
determining, based on the boundary of the object, a plurality of sub-images within the boundary;
resizing to a dimension, based on the boundary having maximal width and maximal length within a determined ratio, at least one sub-image of the plurality of sub-images, wherein resizing comprises downsampling the at least one sub-image;
determining, for each sub-image of the plurality of sub-images, a predicted disease state; and
determining, based on the predicted disease states for each sub-image of the plurality of sub-images, a predicted disease state for the object.

2. The computer implemented method of claim 1, further comprising:
determining, based on the predicted disease states for each sub-image of the plurality of sub-images, a visual indication for each sub-image of the plurality of sub-images;
determining, based on the visual indications for each sub-image of the plurality of sub-images and the image data, a composite image; and
outputting the composite image,
wherein the visual indication changes a hue of each of the one of the sub-images that is weighted according to a first sub-image and a second sub-image of the plurality of sub-images.

3. The computer implemented method of claim 2, wherein the first sub-image is a first 64-pixel-by-64- pixel contiguous block and the second sub-image is a second 64-pixel-by-64-pixel contiguous block.

4. The computer implemented method of claim 2, wherein a fourth of the first sub-image is equal to a first fourth of the second sub-image.

5. The computer implemented method of claim 4, wherein the plurality of sub-images includes a third sub-image, and a second fourth of the first sub-image is equal to the third sub-image and the first fourth and the second fourth overlap.

6. The computer implemented method of claim 1, wherein the dimension is 384 pixels by 384 pixels.

7. The method of claim 2, wherein the first sub-image comprises all pixels within the boundary.

8. The method of claim 1, wherein the first classifier is based on a neural network comprising a residual convolution, an encoder, and a decoder.

9. The method of claim 1, further comprising identifying, by an object identifier, that the object is a polyp.

10. The method of claim 1, further comprising classifying the object as one of malignant or benign.

11. The method of claim 2, wherein determining the visual indication for each sub-image of the plurality of sub-images further comprises determining a color from a plurality of colors associated with the disease state.

12. The method of claim 11, wherein each color of the plurality of colors is associated with a unique disease state of a plurality of disease states.

13. The method of claim 11, further comprising altering, based on the determined color associated with the disease state, a color composition of the pixels within at least one sub-image of the plurality of sub-images.

14. An apparatus, comprising:
one or more processors; and
memory storing processor executable instructions that, when executed by the one or more processors, cause the apparatus to perform the method of any of claims 1-13.

15. One or more non-transitory computer-readable media storing processor-executable instructions that, when executed by at least one processor, cause the at least one processor to perform the method of any of claims 1-13.

## Patentansprüche

1. Ein computerimplementiertes Verfahren, das Folgendes umfasst:
Bestimmen eines Objekts in Bilddaten auf der Grundlage eines ersten Klassifikators;
Ermitteln einer Begrenzung des Objekts auf der Grundlage des Objekts;
Ermitteln einer Vielzahl von Teilbildern innerhalb der Begrenzung auf der Grundlage der Begrenzung des Objekts;
Anpassen der Größe mindestens eines Teilbilds aus der Vielzahl von Teilbildern auf eine Abmessung auf der Grundlage der Begrenzung, die eine maximale Breite und eine maximale Länge innerhalb eines festgelegten Verhältnisses aufweist, wobei das Anpassen der Größe ein Downsampling des mindestens einen Teilbilds umfasst;
Ermitteln eines vorhergesagten Krankheitszustands für jedes Teilbild der mehreren Teilbilder; und
Ermitteln eines vorhergesagten Krankheitszustands für das Objekt auf der Grundlage der vorhergesagten Krankheitszustände für jedes Teilbild der mehreren Teilbilder.

2. Das computerimplementierte Verfahren nach Anspruch 1, das ferner umfasst:
Ermitteln eines visuellen Hinweises für jedes Teilbild der Vielzahl von Teilbildern auf der Grundlage der vorhergesagten Krankheitszustände für jedes Teilbild der Vielzahl von Teilbildern;
Ermitteln eines zusammengesetzten Bildes auf der Grundlage der visuellen Hinweise für jedes Teilbild der Vielzahl von Teilbildern und der Bilddaten; und
Ausgeben des zusammengesetzten Bildes,
wobei der visuelle Hinweis einen Farbton jedes der Teilbilder verändert, das entsprechend einem ersten Teilbild und einem zweiten Teilbild der Vielzahl von Teilbildern gewichtet wird.

3. Das computerimplementierte Verfahren nach Anspruch 2, wobei das erste Teilbild ein erster zusammenhängender Block mit einer Größe von 64 × 64 Pixeln ist und das zweite Teilbild ein zweiter zusammenhängender Block mit einer Größe von 64 × 64 Pixeln ist.

4. Das computerimplementierte Verfahren nach Anspruch 2, wobei ein Viertel des ersten Teilbildes einem ersten Viertel des zweiten Teilbildes entspricht.

5. Das computerimplementierte Verfahren nach Anspruch 4, wobei die Vielzahl von Teilbildern ein drittes Teilbild umfasst und ein zweites Viertel des ersten Teilbildes dem dritten Teilbild entspricht und sich das erste Viertel und das zweite Viertel überlappen.

6. Das computerimplementierte Verfahren nach Anspruch 1, wobei die Abmessung 384 Pixel mal 384 Pixel beträgt.

7. Das Verfahren nach Anspruch 2, wobei das erste Teilbild alle Pixel innerhalb der Begrenzung umfasst.

8. Das Verfahren nach Anspruch 1, wobei der erste Klassifikator auf einem neuronalen Netzwerk basiert, das eine Residual-Faltung, einen Encoder und einen Decoder umfasst.

9. Das Verfahren nach Anspruch 1, das ferner das Identifizieren anhand eines Objektidentifikators umfasst, dass es sich bei dem Objekt um einen Polypen handelt.

10. Das Verfahren nach Anspruch 1, das ferner das Klassifizieren des Objekts als bösartig oder gutartig umfasst.

11. Das Verfahren nach Anspruch 2, wobei das Bestimmen des visuellen Hinweises für jedes Teilbild der Vielzahl von Teilbildern ferner das Bestimmen einer Farbe aus einer Vielzahl von Farben umfasst, die dem Krankheitszustand zugeordnet sind.

12. Das Verfahren nach Anspruch 11, wobei jede der mehreren Farben einem eindeutigen Krankheitszustand aus einer Vielzahl von Krankheitszuständen zugeordnet ist.

13. Das Verfahren nach Anspruch 11, das ferner das Ändern einer Farbzusammensetzung der Pixel innerhalb mindestens eines Teilbildes der Vielzahl von Teilbildern auf der Grundlage der ermittelten, dem Krankheitszustand zugeordneten Farbe umfasst.

14. Eine Vorrichtung, umfassend:
einen oder mehrere Prozessoren; und
einen Speicher, der prozessorausführbare Befehle speichert, die, wenn sie von dem einen oder den mehreren Prozessoren ausgeführt werden, bewirken, dass die Vorrichtung das Verfahren gemäß einem der Ansprüche 1-13 ausführt.

15. Ein oder mehrere nichtflüchtige, computerlesbare Speichermedien, auf denen prozessorausführbare Befehle gespeichert sind, die, wenn sie von mindestens einem Prozessor ausgeführt werden, bewirken, dass der mindestens eine Prozessor das Verfahren gemäß einem der Ansprüche 1 bis 13 ausführt.

## Revendications

1. Procédé mis en œuvre par ordinateur, comprenant:
la détermination, à l'aide d'un premier classificateur, d'un objet dans des données d'image;
la détermination, sur la base de l'objet, d'un contour de l'objet ;
la détermination, sur la base du contour de l'objet, d'une pluralité de sous-images situées à l'intérieur dudit contour ;
le redimensionnement, à une dimension déterminée en fonction du contour présentant une largeur maximale et une longueur maximale respectant un rapport prédéfini, d'au moins une sous-image parmi la pluralité de sous-images, le redimensionnement comprenant un sous-échantillonnage de ladite au moins une sous-image ;
la détermination, pour chaque sous-image de la pluralité de sous-images, d'un état pathologique prédit ; et
la détermination, à partir des états pathologiques prédits pour chaque sous-image de la pluralité de sous-images, d'un état pathologique prédit pour l'objet.

2. Procédé mis en œuvre par ordinateur selon la revendication 1, comprenant en outre:
la détermination, sur la base des états pathologiques prédits pour chaque sous-image de la pluralité de sous-images, d'une indication visuelle pour chaque sous-image de la pluralité de sous-images;
la détermination, sur la base des indications visuelles pour chacune des sous-images de la pluralité de sous-images et des données d'image, d'une image composite ; et
la génération de l'image composite,
dans lequel l'indication visuelle modifie la teinte de chacune des sous-images pondérées en fonction d'une première sous-image et d'une deuxième sous-image de la pluralité de sous-images.

3. Procédé mis en œuvre par ordinateur selon la revendication 2, dans lequel la première sous-image est un premier bloc contigu de 64 pixels sur 64 pixels et la deuxième sous-image est un deuxième bloc contigu de 64 pixels sur 64 pixels.

4. Procédé mis en œuvre par ordinateur selon la revendication 2, dans lequel un quart de la première sous-image est égal à un premier quart de la deuxième sous-image.

5. Procédé mis en œuvre par ordinateur selon la revendication 4, dans lequel la pluralité de sous-images comprend une troisième sous-image, et un deuxième quart de la première sous-image est égal à la troisième sous-image, et le premier quart et le deuxième quart se chevauchent.

6. Procédé mis en œuvre par ordinateur selon la revendication 1, dans lequel la dimension est de 384 pixels sur 384 pixels.

7. Procédé selon la revendication 2, dans lequel la première sous-image comprend tous les pixels situés à l'intérieur du contour.

8. Procédé selon la revendication 1, dans lequel le premier classificateur est basé sur un réseau neuronal comprenant une convolution résiduelle, un codeur et un décodeur.

9. Procédé selon la revendication 1, comprenant en outre l'identification, à l'aide d'un module d'identification d'objet, du fait que l'objet est un polype.

10. Procédé selon la revendication 1, comprenant en outre la classification de l'objet comme étant soit malin, soit bénin.

11. Procédé selon la revendication 2, dans lequel la détermination de l'indication visuelle pour chaque sous-image de la pluralité de sous-images comprend en outre la détermination d'une couleur parmi une pluralité de couleurs associées à l'état pathologique.

12. Procédé selon la revendication 11, dans lequel chaque couleur parmi la pluralité de couleurs est associée à un état pathologique unique parmi une pluralité d'états pathologiques.

13. Procédé selon la revendication 11, comprenant en outre la modification, sur la base de la couleur déterminée associée à l'état pathologique, d'une composition chromatique des pixels au sein d'au moins une sous-image parmi la pluralité de sous-images.

14. Appareil, comprenant :
un ou plusieurs processeurs ; et
une mémoire stockant des instructions exécutables par un processeur qui, lorsqu'elles sont exécutées par le ou les processeurs, amènent l'appareil à mettre en œuvre le procédé selon l'une quelconque des revendications 1 à 13.

15. Un ou plusieurs supports non transitoires lisibles par ordinateur stockant des instructions exécutables par un processeur qui, lorsqu'elles sont exécutées par au moins un processeur, amènent ledit au moins un processeur à mettre en œuvre le procédé selon l'une quelconque des revendications 1 à 13.
